# EUROPEAN PATENT APPLICATION

(11) **EP 4 227 399 A1**
(43) Date of publication of application: **16.08.2023**
(21) Application number: 22382119.0
(22) Date of filing: 14.02.2022
(51) Int. Cl.: C12M 3/00, A61L 27/38, C12M 1/26, C12N 5/079

(54) **ARTIFICIAL CONSTRUCTS FOR USE IN OPHTHALMOLOGY, METHODS OF OBTAINING THE SAME, AND THEIR USE**

(71) Applicant: Fundación para la Investigación Biomédica del Hospital Universitario de la Paz, 28046 Madrid (ES); Fundación Para La Investigación Biomédica Del Hospital Universitario Ramón Y Cajal (FIBIOHRC), 28034 Madrid (ES)
(72) Inventor: DE MIGUEL GONZÁLEZ, Maria de la Paz, 20046 Madrid (ES); ARNALICH MONTIEL, Francisco, 28034 Madrid (ES); MORATILLA RIOFRÍO, Adrián, 28034 Madrid (ES); CADENAS MARTÍN, Marta, 20046 Madrid (ES)
(74) Representative: Hoffmann Eitle

(57) **Abstract**

Procedure for obtaining an artificial corneal construct by 3D printing. The procedure comprises the following stages: i. providing a composition in the form of bioink comprising type I collagen or a mixture of type I, type V and/or type VI collagen at a concentration between 0.2 and 80 mg/ml, at a pH between 3.4 and 9 and where the composition optionally comprises proteoglycans of corneal origin selected from the group consisting of chondroitin sulfate, dermatan sulfate and keratan sulfate or any combination thereof at a concentration between 1 and 10 µg/ml; ii. printing between 1 and 25 successive layers of the composition comprising the bioink from the previous stage by means of 3D printing, wherein 3D printing is characterized by presenting an accumulated height of between 200 and 6000 µm and producing each layer to obtain collagen fibrils having a direction with an angle of 70° to 110° with respect to the direction of the collagen fibrils of the previous layer, keeping the bioink at a temperature that does not gel; iii. Incubating the layered product obtained from stage ii at 30-40 °C to induce collagen gelation; and iv, incubating the layered product obtained from stage iii at a temperature of 20-40 °C and a humidity of 10 to 90% to induce its compaction, hardening and/or controlled dehydration until the succession of layers comprises a size between 20 and 600 µm, with a thickness per layer between 20 and 25 µm thick.

## Description

### Field of the invention

The present invention refers to the procedure for obtaining an artificial corneal construct, to the corneal construct obtained or obtainable through the process, to its use and to a computer program capable of controlling a 3D printer to print a corneal construct according to the procedure, finding its application mainly in the field of ophthalmology.

### Background of the invention

Currently, diseases of the cornea are one of the main causes of vision loss, affecting more than 15 million people worldwide. Ocular trauma and corneal ulceration due to infection are important causes of corneal blindness that often go unreported but may be responsible for 1.5 - 2 million new cases of monocular blindness each year.

Specifically, among the pathologies of the corneal stroma, the following can be pointed out:
A) Inflammatory pathologies, such as severe conjunctivitis with secondary involvement of the corneal stroma (trachoma, pemphigoid, atopic keratoconjunctivitis, graft-versus-host disease, Stevens-Johnson syndrome, vernal keratoconjunctivitis, phlyctenular keratoconjunctivitis, blepharoconjunctivitis in all its forms, etc.), viral infectious keratitis (herpes simplex virus, herpes zoster virus, adenovirus), bacterial, (fungal and amoebic), interstitial keratitis (syphilitic and non-syphilitic (Cogan syndrome, Lyme disease, tuberculosis, HSV/VHZ, sarcoidosis, lymphomas)), keratitis secondary to autoimmune diseases (connectivopathies (rheumatoid arthritis, Sjogren), sarcoidosis, idiopathic (Mooren's ulcer), etc.), corneal trauma (chemical burns, inflammation associated with foreign bodies, iatrogenesis, etc.).
B) Non-inflammatory pathologies, such as corneal dystrophies, corneal ectasias (keratoconus, keratoglobus, pellucid marginal degeneration, Terrien's marginal degeneration, post-LASIK ectasia), corneal degenerations (Salzmann's nodular degeneration, band keratopathy), neurotrophic keratopathy, factitious keratoconjunctivitis (abuse of topical anesthetics) or neoplasms (corneal CIN, choristomas, lymphomas).

Currently, the treatment of all of these conditions may require a corneal transplant from a human donor. This transplant has an average success rate of 80%, depending on the pathology and condition of the host. Non-inflammatory pathologies have a low risk of rejection, but worldwide the demand for donor corneas far exceeds the supply. On the other hand, the aging of the population and the longer life expectancy means that donations of quality decrease (corneas are getting older), and more are needed (the longer the life expectancy, the more chances of needing a transplant). The increase in refractive surgery may aggravate these deficiencies in the coming years, since, to date, corneas treated with laser are not considered suitable for transplantation that requires replacement of the corneal stroma.

These accessibility and supply problems highlight the need for the creation of an artificial cornea that can replace the natural cornea from a donor in the future.

The generation of a prosthesis similar to natural stromal tissue in terms of strength and transparency, capable of being efficiently repopulated by the patient's own cells and of adequately biointegrating without producing rejection or inflammatory reaction and with biomechanics similar to that of natural stromal tissue, would represent a great advance in the treatment of patients who require a corneal transplant due to pathologies associated with the stroma and/or who are considered to be at high risk of rejection.

In addition, in the case of transplantation in inflammatory pathologies, including previous corneal rejections, it is normally associated with a high rate of rejection. For these cases of high risk of rejection, including multiple failures of previous grafts, advances in research that allow the development of new biomaterials with better biointegration in the corneal stroma are required.

To date, the alternatives to human donor cornea transplantation used include artificial substitutes (keratoprosthesis), which are already used to restore vision in cases of severe ocular surface pathology and after transplant rejection.

Although many keratoprosthesis models have been proposed in recent years, only two of them are currently implanted to a significant extent in America and Europe: the osteo-odonto-keratoprosthesis and the Boston keratoprosthesis. These devices have limited indications for their use, since none of the existing prostheses integrate perfectly into the host tissue, and a retrocorneal membrane may occur that eventually causes extrusion of the prosthesis, high risk of intraocular infection and endophthalmitis, or the development or aggravation of glaucoma.

Alternatively, in recent years approaches have been used with Cell Therapy methods, that is, the transplantation of cells that secrete extracellular matrix similar to the corneal stroma in sufficient quantities to regenerate corneal damage. The possibility of producing corneal stroma from the stromal stem cell, already characterized, has been proposed, but neither the stromal stem cells nor the differentiated keratocytes are numerous or easy to extract without damaging the donor's cornea, which makes it difficult to perform an autologous transplant of the healthy eye. An extraocular source of autologous stem cells is therefore required.

In previous studies, this problem was addressed with a Cell Therapy approach using an extraocular source: adult adipose-derived mesenchymal stem cells (ADSC) first by cell transfer exclusively. The present inventors demonstrated that ADSCs differentiated *in vivo* to corneal stromal cells and produced the appropriate collagens and proteoglycans, but in small amounts. Next, we produced keratoprostheses using ADSC-coated polyethylacrylate-type biomaterials. Although this approach improved graft survival, graft extrusion invariably occurred. The most promising approach, also previously developed by the present inventors, has been the use of donor human corneal sheets, which have been decellularized to avoid rejection and later recellularized with ADSC, where complete integration and transparency, added to stability of the implant were achieved. However, while this approach allows one donor cornea to be used for multiple patients, and in fact has led to the promotion of a phase II clinical trial using autologous ADSCs, it still does not provide an unlimited source of corneal prostheses for use in patients as it still needs tissue from a donor.

Recent studies have demonstrated a proof of concept for the use of biomimetic materials (with both composition and structure similar to corneal stroma) as corneal implants to promote corneal regeneration as an alternative to donor cornea implantation in rabbits (Liu Y., et al., Griffith M. A simple, cross-linked collagen tissue substitute for corneal implantation. Invest Ophthalmol Vis Sci. 2006, 47(5), 1869-1875.15). Implants were made from cross-linked collagen hardened with carbodyimide, with optical and chemical properties similar to human corneas. When used as cornea substitutes for transplantation, the implants stimulated the regeneration of corneal tissue in experimental animals, without the need for immunosuppression. However, these implants had significantly weaker tensile strengths compared to the human cornea (Merrett K., et al., Griffith M. Tissue-engineered recombinant human collagen-based corneal substitutes for implantation: performance of type I versus type III collagen. Invest Ophthalmol Vis Sci. 2008, 49(9), 388738-94). Recently, a development of 3D printing for the corneal stroma has been carried out (Isaacson A, Swioklo S, Connon CJ (2018). 3D bioprinting of a corneal stroma equivalent. Exp Eye Res, 173:188-193; Duarte Campos, DF, et al., Fuest, M., (2019). Corneal bioprinting utilizing collagen-based bioinks and primary human keratocytes. J. Biomed. Mater. Res. 107 (9), 1945-1953), but the results obtained, which are only *in vitro* results, do not reveal the transparency and strength of these constructs.

In document CN105034369, "Three - dimensional (3D) cornea stroma support material and method for constructing three-dimensional cornea stroma support", a three-dimensional (3D) cornea stroma support material and a corresponding method are disclosed. The material comprises 1%-5% chitosan, 5%-15% collagen, 5%-15% polyvinyl alcohol, and water up to 100%. The method includes the steps for establishing a computer model of the corneal stromal support in 3D and defining print files; preparing a solution of the 3D corneal stromal support material to be used as printing ink and printing successive layers to form a printed support, which is lyophilized, cross-linked, cleaned and disinfected. This approach is not biomimetic in composition and has not been tested *in vivo* to demonstrate biocompatibility.

WO 2016049345 A1 describes a method of manufacturing an artificial cornea from a culture of stromal cells and living corneal endothelial cells that are encapsulated in two hydrogels by 3D printing and that are finally united into a single corneal structure. This method, like the previous one, proposes a non-biomimetic approach, using a plastic pre-polymeric solution whose medium-term rejection is foreseeable, as occurs with the current keratoprostheses explained above.

There is therefore a need to provide a biomimetic artificial corneal construct, with a composition and structure similar to the corneal stroma, which allows such transparency that it allows the passage of light towards the retina, with a low rejection rate and a simple and rapid production that meet the high demand for these constructs.

### Summary of the invention

A first aspect of the invention refers to a process for obtaining an artificial corneal construct by three-dimensional (3D) printing, which comprises the following stages:
i. providing a composition in the form of bioink, comprising type I collagen or a mixture of type I, type V and/or type VI collagen at a concentration between 0.2 and 80 mg/ml, at a pH between 3.4 and 9. In addition, the composition optionally comprises proteoglycans of corneal origin selected from the group consisting of chondroitin sulfate, dermatan sulfate and keratan sulfate or any combination thereof, at a concentration of between 1 and 10 µg/ml;
ii. printing between 1 and 25 successive layers of the composition comprising the bioink from the previous stage by 3D printing. 3D printing is also characterized by presenting an accumulated height of between 200 and 6000 µm, and by producing each layer to obtain collagen fibrils having a direction with an angle of 70° to 110° with respect to the direction of the collagen fibrils of the layer above, keeping the bioink at a temperature that does not gel.
iii. incubating the layered product obtained from the previous stage at 30-40 °C to induce collagen gelation; and
iv. incubating the layered product obtained from the previous stage at a temperature of 20 - 40 °C and a humidity of 10 to 90% to induce its compaction, hardening and/or controlled dehydration until the succession of layers comprises a size between 20 and 600 µm, with a thickness per layer between 20 and 25 µm thick.

In one embodiment, the process of obtaining further comprises the hydration of the hardened product of stage iv in a water-based solution, preferably in a buffer solution.

In a preferred embodiment according to any of the previous embodiments, stage ii 3D printing is further characterized by having a print stream diameter of between 125 and 900 µm, and/or a print speed between 0.5 and 1600 mm/s, and/or a pore size between 100 and 600 µm and/or a variation in height per layer of at least ten times the thickness per layer of stage iv. The diameter of the printing stream is understood as the diameter of the bioink at each point of bioink deposition; the printing speed is understood as the horizontal speed at which a 3D printer produces the construct at each point, the pore size is understood as the distance between adjacent printed lines of a layer that generate a free space of bioink between layers, and the variation in height per layer is understood as the distance at which a layer occurs over the previous layer. In an even more preferred embodiment, the 3D printing is performed by extrusion and the diameter of the nozzle corresponds to the diameter of the printing stream. Optionally, the 3D printing is also characterized by a retraction speed of between 5 and 60 mm/s; and/or by a running speed of between 30 and 70 mm/s and/or by a printing speed of between 0.5 and 700 mm/s.

In another preferred embodiment according to any of the previous embodiments, the composition further comprises keratocytes and/or cells capable of differentiating into keratocytes. Cells that can differentiate into keratocytes may be corneal fibroblasts, stromal fibroblasts, fibroblasts of any origin, stromal stem cells, mesenchymal stem cells of any origin, including adipose-derived stem cells (ADSC), corneal stromal cells, multipotent stem cells of any origin, embryonic stem cells, pluripotent cells of any origin including induced pluripotent stem cells, mesenchymal cells of any origin, mesothelial cells, limbal stem cells, neural crest cells and/or mesenchymal stem cells. More preferably, the keratocytes and/or cells capable of differentiating to keratocytes are present in the composition at a concentration in the range of 1 · 10 ⁵ - 6 · 10 ⁵ cells/mm^{3,} preferably in the range of 3 · 10 ⁵ - 5 · 10 ⁵ cells/mm³.

In an even more preferred embodiment according to any of the previous embodiments, the collagen is 100% type I collagen or a mixture of type I, type V and/or type VI collagen in a proportion of 81% +/- 10%., 17% +/- 10% and 2% +/- 10%, respectively.

In another preferred embodiment according to any of the previous embodiments, the thickness per final layer is +/- 30% at 20 µm thickness, and the 3D printing is characterized by a diagonal loading pattern of approximately 90°, while maintaining the bioink at a temperature between 2°C and 25°C or between 50°C and 60°C, preferably at 4°C. Optionally, 3D printing is further characterized by a nozzle print stream diameter of +/- 30% of 580 microns, a print speed of +/- 30% of 3 mm/s, a pore size of +/- 30 % of 300 µm, a variation in height per layer of +/- 30% of 1 mm, a retraction speed of +/- 30% of 5 mm/s and/or a running speed of +/- 30% of 40 mm/sec. In other even more preferred embodiments, the variation of these ranges is +/- 20%, +/- 10%, +/- 5% and +/- 1%.

A second aspect of the invention refers to an artificial corneal construct obtainable or obtained according to any of the methods of obtaining of the first aspect of the invention. In a preferred embodiment, the construct is obtained or obtainable in the form of a rectangular parallelepiped or cylinder, where the base of the rectangular parallelepiped and the base of the cylinder have a side and diameter, between 1 and 20 mm respectively, more preferably between 1 and 13 mm, even more preferably between 8 and 10mm.

A third aspect of the invention relates to the artificial corneal construct according to the second aspect of the invention for its use in therapy.

In a preferred embodiment where the construct comprises a thickness of 100 to 150 microns, it refers to its use in methods of treating ocular pathologies such as keratoconus, Terrien's marginal degeneration, pellucid marginal degeneration, Mooren's ulcer, corneal thinning, corneal burns, in pterygium surgery, perforation of the sclera or cornea, and strabismus; in limbal transplantation treatments such as limbal epithelial cell culture transplants (CLET) and simple limbal epithelial cell transplantation (SLET), as a substitute for amniotic membrane in corneal epithelial surgeries or anterior lamellar keratoplasty and as a support or vehicle for corneal endothelium in keratoplasty surgeries such as Descemet's membrane stripping endothelial keratoplasty (DSAEK) and/or Descemet's membrane endothelial keratoplasty (DMEK) among others.

In another preferred embodiment where the construct comprises a thickness of 100 to 150 microns, it refers to its use in methods of treating ocular pathologies such as stromal transplant, complete or penetrating corneal transplant (400 to 600 microns) or correction of ametropias.

A fourth aspect of the invention refers to a computer program, connected to a 3D printer. The computer program comprises computer-readable instructions that, when executed by a processor, cause the processor to execute the step of printing between 1 and 25 successive layers of a composition using 3D printing. The composition in the form of a bioink comprises type I collagen or a mixture of type I, type V and/or type VI collagen at a concentration between 0.2 and 80 mg/ml, at a pH between 3.4 and 9. The composition optionally comprises proteoglycans of corneal origin selected from the group consisting of chondroitin sulfate, dermatan sulfate and keratan sulfate or any combination thereof at a concentration of between 1 and 10 µg/ml. 3D printing is characterized by presenting an accumulated height between 200 and 6000 µm and producing each layer to obtain collagen fibrils having a direction with an angle of 70° to 110° with respect to the direction of the collagen fibrils of the previous layer, keeping the bioink at a temperature that does not gel.

### Brief description of the figures

To enable a better understanding of the present disclosure, and to show how the same may be carried out, reference will now be made, by way of example only, to the accompanying schematic drawings, in which:
- Figure 1:: Photographs showing the *in vitro* transparency of the artificial corneal construct at different stages of the obtaining procedure according to one or more embodiments. Figures 1A and 1B show the construct after gelation. Figures 1C and 1D show the construct after dehydration. Figures 1E and 1F show the construct after rehydration.
- Figure 2:: Measurements of thickness by optical coherence tomography (OCT) *in vitro* of an artificial corneal construct according to one or more embodiments of the invention.
- Figure 3:: Photographs showing the *in vitro* biocompatibility of an artificial corneal construct according to one or more embodiments of the invention with keratocytes on days 1 and 7 of culture.
- Figure 4:: Photographs showing targeted differentiation of adipose-derived stem cells into keratocytes on a corneal construct according to one or more embodiments of the invention, phase contrast (Fig. 4A) by fluorescence staining of the nucleus (Fig. 4B) and immunofluorescence, showing the secretion of keratocan (FIG. 4C) and collagen IV (Fig. 4D) 21 days after differentiation.
- Figure 5:: Photographs showing *in vivo* biocompatibility and safety of a corneal construct according to one or more embodiments of the invention in transplanted rabbit eyes over time.
- Figure 6:: Image of a cornea transplanted (in rabbits) with an artificial corneal construct according to one or more embodiments of the invention taken by OCT, showing its complete biointegration.
- Figure 7:: Histological image of the cornea showing a corneal construct according to one or more embodiments of the invention transplanted (3D) and biointegrated in the corneal stroma (Str).
- Figure 8:: Diagram of the procedure for obtaining an artificial corneal construct by 3D printing according to one or more embodiments.
- Figure 9:: Photographs showing the *in vitro* transparency of an artificial corneal construct at different stages of the obtaining procedure according to one or more embodiments. Figure 9A shows the construct after gelation. Figure 9B shows the construct after dehydration. Figure 9C shows the construct after rehydration. Figure 9D shows the construct in glycerol.
- Figure 10:: Photographs showing the *in vitro* transparency of another artificial corneal construct at different stages of the obtaining procedure according to one or more embodiments. Figure 10A shows the construct after gelation. Figure 10B shows the construct after dehydration. Figure 10C shows the construct after rehydration. Figure 10D shows the construct in glycerol.
- Figure 11:: Photographs showing the *in vitro* transparency of another artificial corneal construct at different stages of the obtaining procedure according to one or more embodiments. Figure 11A shows the construct after gelation. Figure 11B shows the construct after dehydration. Figure 11C shows the construct after rehydration. Figure 11D shows the construct in glycerol.
- Figure 12:: Photographs showing the *in vitro* transparency of another artificial corneal construct at different stages of the obtaining procedure according to one or more embodiments. Figure 12A shows the construct after gelation. Figure 12B shows the construct after dehydration. Figure 12C shows the construct after rehydration. Figure 12D shows the construct in glycerol.
- Figure 13:: Product diagram of computer program according to one or more embodiments.
- Figure 14:: Shows a schematic view of the deposition direction of two construct layers according to one or more embodiments.
- Figure 15:: Profile of a construct in its 3D printing stage ii) according to one or more embodiments. Figures 15A-C show the 3D printing process of a construct at different time points. Figure 15D shows another construct according to one or more embodiments.
- Figure 16:: Diagram of a bioink extrusion system according to one or more embodiments.
- Figure 17:: Shows a plan (Figure 17A) and perspective view (Figure 17B) of the pore size of a construct according to one or more embodiments.
- Figure 18:: Cross-sectional sample of the human corneal stroma that the construct is mimicking according to one or more embodiments.

### Detailed description of the invention

### Definitions:

The term "3D printing" or "three-dimensional printing" refers to an additive-type manufacturing procedure, in which a material is produced, generating an object in three dimensions. There are several types of 3D printing. Thus, for example, printing by extrusion of material deposits the material through a nozzle at different points at various heights to form the object. Several subtypes can be defined: by pneumatic force, by mechanical piston or by screw extruder. Jet printing induces the formation of drops of material by temperature, laser or piezoelectricity in deposited droplets. In turn, it can be divided into the subtypes: "laser-induced forward transfer" (LIFT), micro-valve or "inkjet" printing. Vat photopolymerization printing uses light to cure a resin. Several subtypes are defined such as digital light projection, stereolithography, and two-photon polymerization. It is noteworthy that other 3D printing methods not described can be used within this invention, and therefore the invention is not limited to the 3D printing method types herein described, but to all those with the ability to print with the parameters defined in each realization.

The verb "to deposit" refers to the provision of a certain amount of material at each point of a layer by a 3D printer. Thus, in some types of printers, the material can be deposited by gravity or by a mechanical system (such as an extruder nozzle), while in others it can be done by curing a resin. In embodiments where the term "deposit" is used it is understood that in alternative embodiments the construct may be provided by any other printing means disclosed herein. Thus, for vat photopolymerization, the construct can be provided by curing the bioink.

The term "layers" refers to the vertically stacked sections that are produced by the 3D printer to form the printed object. The number of layers of an object refers to the number of times that a three-dimensional object is divided in a certain direction for 3D printing, corresponding to each of the heights that a 3D printer defines to produce the construct at each point, at one layer for each height. In artificial corneal construct prints, the layers are preferably defined in the direction perpendicular to the plane defined by the cornea when the artificial cornea is in an implanted configuration.

The term "bioink" refers to 3D printing material in biological applications, where the bioink comprises some biological element, be it cells, their precursors or organic elements such as collagen. It is normally found in liquid or gel format to facilitate layering. It can have a range of characteristics, such as viscosity, surface tension, and density, characteristics optimized with composition, concentration, pH, and temperature.

The term "thickness per layer" refers to the thickness of the material produced per layer at a given point by a 3D printer. The thickness per layer can be defined at the time of deposition, called "initial layer thickness", or once it is cured or treated to provide better physical characteristics, called "final layer thickness". Thus, the thickness per layer can be increased or decreased in the curing and/or treatment process. In extruder printers it corresponds to the printing height, in laser jet printers it depends on the impact speed, and in vat photopolymerization printers it depends on the optical absorbance of the resin and the viscosity of the photopolymer.

The term "print stream diameter" refers to the diameter of the bioink at each point of bioink deposition by the 3D printer. In extrusion printers it is determined by the diameter of the extrusion nozzle, in the case of laser jet printers by the pulse frequency and the size of the laser beam, and in the case of vat photopolymerization printers by the laser beam size and optical fluence. Specifically, the diameter of the print stream defines the print resolution of the layer in the horizontal direction.

The term "print speed" refers to the horizontal speed at which a 3D printer produces the construct at each point. It is measured in distance by time. In extrusion printing it depends on the material flow rate, in jet printers it depends on the pulse frequency, and in vat photopolymerization on the optical fluence and scanning speed.

The term "substrate" refers in laser jet printers to the medium on which the bioink droplets are deposited. Thus, each layer is formed on the substrate by deposition of bioink droplets that will be joined together by coalescence depending on the wettability of each droplet.

The term "surface" refers, in vat photopolymerization printers, to the resin or polymer in which the bioink droplets in each layer are polymerized. Thus, each layer is formed on the surface by photopolymerization.

The term "fill rate" refers to the proportion of the volume of bioink that will comprise the interior of the corneal construct, relative to the total volume of the corneal construct, defined between 0% (hollow) and 100% (solid).

The term "pore size" refers to the distance between adjacent printed lines within a layer that generates a bioink-free space between printed lines within a layer. It is inversely related to the fill rate. In laser jet printing it depends on the wettability of the substrate and in vat photopolymerization printing depends on the surface coating.

The term "fill pattern" refers to the angle between the directions of the bioink depositions and therefore of the collagen fibrils between two adjacent layers. The fill pattern of a second layer produced on a first layer refers to the angle between the collagen fibrils of the first layer and the second layer. Thus, a 90° fill pattern indicates that each layer is printed in a direction perpendicular to the previous one.

The term "retraction speed" refers, in extrusion printers, to the speed at which the nozzle extruder reverses the sense of ink deposition within the nozzle to avoid unintentionally depositing bioink on nozzle movements where printing is not desired.

The term "perimeter velocity" refers to the horizontal nozzle speed at which the construct is produced at the perimeter of the desired shape in each layer.

The term "fill rate" refers to the horizontal nozzle rate at which the construct is produced within the perimeter of each layer.

The term "running speed" refers, in extrusion printers, to the horizontal speed of the print nozzle when it is not ejecting bioink.

The term "height variation per layer" refers to the distance at which one layer is produced over the previous layer. In extruder and jet printers it corresponds to the height of the nozzle with respect to the previous layer, and in vat photopolymerization type printers corresponds to the displacement of the cured layers of liquid resin from layer to layer.

The term "media preparator" refers to a device capable of selecting, manipulating and mixing a series of given media to form one or more of the compositions described in the present invention.

The term "incubation chamber" refers to a device capable of maintaining desired environmental conditions such as temperature and humidity for a predetermined period of time for the incubation of biological samples.

The term "strength" refers to the ability of the construct to resist applied stresses and forces without breaking, acquiring permanent deformation, or deteriorating in any way.

The term "hardness" refers to the ability of the construct to resist penetration, scratching, and/or abrasion.

The term "elasticity" refers to the ability of the construct to reverse the deformation suffered when an external force is applied once the external force is removed.

In the context of the present invention, the comma has been used as an orthographic sign to separate decimals.

### Description

The present invention provides a method for obtaining an artificial construct manufactured by three-dimensional printing (3D), composed of a mixture of collagens and/or proteoglycans that mimics ocular structures such as the native human corneal stroma. This mixture is printed and compacted to increase its strength by different methods, giving rise to a three-dimensional polymeric structure capable of achieving the desired strength, biomechanics and transparency of the imitated ocular structure, such as the strength and transparency of the corneal stroma. This matrix can act as a support for keratocytes and/or cells capable of differentiating into keratocytes, such as mesenchymal stem cells, corneal stromal cells, stromal fibroblasts of any origin, multipotent cells of any origin, embryonic stem cells, pluripotent cells of any origin including induced pluripotent stem cells, mesenchymal cells of any origin, mesothelial cells, stem limbal cells, neural crest cells, and the like, which may or may not be previously differentiated into keratocytes to form the entire corneal stroma.

From now on, reference will be made to the construct that mimics the corneal stroma, the present invention therefore not being limited to corneal stroma constructs. It is therefore understood that artificial constructs for ophthalmological use such as ocular sclera constructs can also be obtained with the procedures described herein.

Thus, the generation of polymeric collagen structures similar to the human stroma by 3D printing, together with their colonization by keratocytes and/or cells capable of differentiating into keratocytes isolated from the patient, provides stable prostheses that do not depend on accessibility to donor tissue.

In a first aspect, the present invention relates to a method for obtaining an artificial corneal construct by 3D printing, which comprises the following stages:
i) Providing a composition in the form of bioink comprising type I collagen or a mixture of type I, type V and/or type VI collagen at a concentration between 0.2 and 80 mg/ml and at a pH between 3.4 and 9. The bioink formed by collagen types I, V and/or VI at different concentrations make up a biomimetic composition as it is composed of the same proteins as the corneal stroma. This provides a lower rate of rejection by the body. The different types of collagen can be arranged at different concentrations, as will be described later, depending on the use. Concentrations between 0.2 and 80 mg/ml have been tested in 3D bioprinting uses. In a preferred embodiment, the concentration is between 6 and 10 mg/ml. In an even more preferred embodiment, the concentration is 8mg/ ml. A pH close to 3.4 or 9 allows the denaturation of collagen for its application as bioink. In a preferred embodiment, the bioink is at a pH of 7.47 and denaturation occurs by temperature, as will be described later.
   The composition optionally comprises proteoglycans of corneal origin selected from the group consisting of chondroitin sulfate, dermatan sulfate and keratan sulfate, or any combination thereof at a concentration between 1 and 10 µg/ml, as will be described later.
ii) Printing between 1 and 25 successive layers of the composition comprising the bioink from the previous stage by 3D printing, where 3D printing is characterized by presenting an accumulated height of at least 200 µm and producing each layer to obtain collagen fibrils having a direction with an angle of 70° to 110° with respect to the direction of the collagen fibrils of the previous layer, keeping the bioink at a temperature that does not gel. In a preferred embodiment, the 3D print is characterized by having a cumulative height of between 200 and 6000 µm.

It should be noted that producing each layer to obtain collagen fibrils having a direction with an angle of 70° to 110° with respect to the direction of the collagen fibrils of the previous layer provides a biomimetic characteristic to the construct, by replicating the arrangement of the different layers of the corneal stroma, close to 90°, which ensures the transparency of the construct. Stromal transparency is primarily a consequence of the order in the arrangement of collagen fibrils and the uniformity of fibril diameter. Light entering the cornea is scattered by each fibril. The arrangement and diameter of the fibrils is such that scattered light constructively interferes only in the forward direction, allowing light to pass to the retina. Thus, producing each layer in a specific direction shapes the collagen fibrils in that direction. The deposition of bioink in each layer to obtain collagen fibrils having a direction with an angle of 70° to 110° with respect to the direction of the collagen fibrils of the previous layer, causes the collagen fibrils of said layer to be in one direction with an angle of 70° to 110° with respect to the direction of the collagen fibrils in the previous layer, thus obtaining the desired transparency.

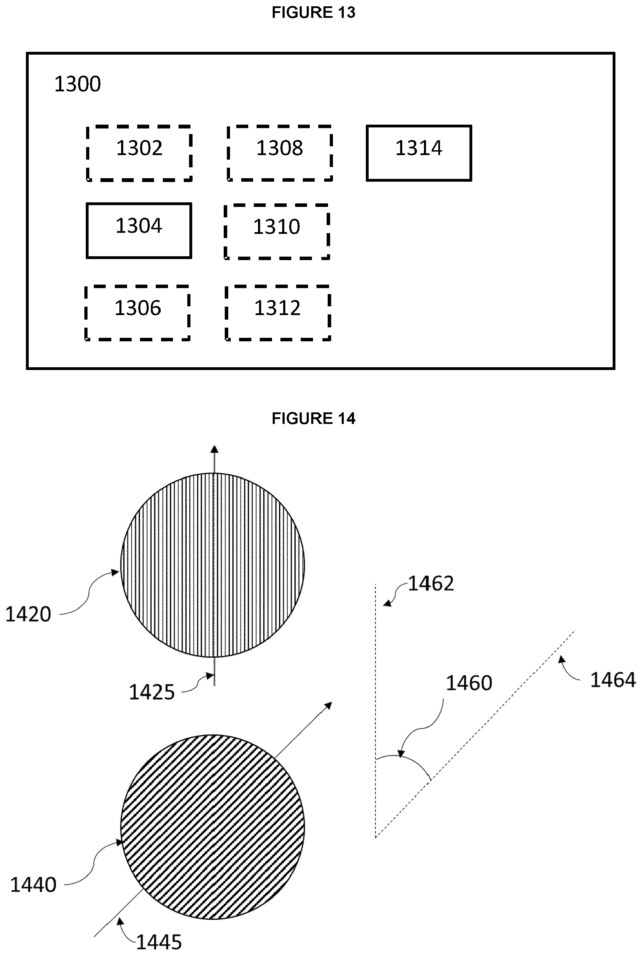

Figure 14 shows a representation of two layers with their deposition directions according to one or more embodiments. A first 3D printed layer 1420 has a first print direction 1425. A second 3D printed layer 1440 has a second print direction 1445. The print angle of the second layer 1440 relative to the first layer 1420 is defined by the angle 1460 formed between straight lines 1462 and 1464, where line 1462 is parallel to the print direction 1425 of the first layer 1420, and line 1464 is parallel to the print direction 1445 of the first layer 1440.

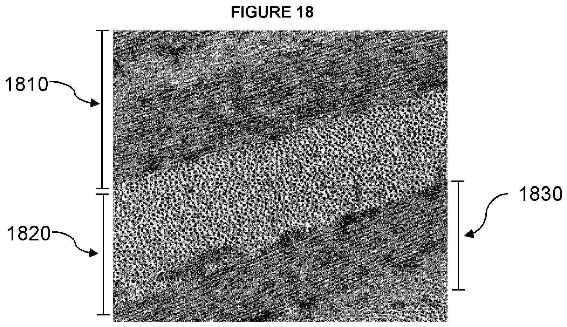

Figure 18 shows a cross-section of the human corneal stroma, showing that the collagen fibrils of the layers are approximately 90° with respect to the direction of the collagen fibrils of the previous layer. Thus, the alternate layers 1810 and 1830 are approximately parallel to each other and at 90° to the anterior and posterior layer respectively 1820. Therefore, producing each layer to obtain collagen fibrils having a direction angled from 70° to 110° with respect to the direction of the collagen fibrils of the anterior layer provides a biomimetic characteristic to the construct.

In other non-corneal artificial constructs, the desired transparency may be different. Thus, alternatively, when the artificial construct produced requires less transparencies, in stage ii) of the process of obtaining the artificial construct, 3D printing can be characterized by producing each layer to obtain collagen fibrils having a direction with an angle between 0° and 359° with respect to the direction of the collagen fibrils of the previous layer. For example, when the artificial construct is made for sclera, 3D printing is characterized by producing each layer to obtain collagen fibrils having a direction with an angle between 315° and 45° with respect to the direction of the collagen fibrils of the previous layer.

It should also be noted that the temperature that does not gel the bioink depends on several factors, such as the type of collagen included and their concentration. In a preferred embodiment, the temperature of the bioink is between 2 and 25°C or between 50 and 60°C, more preferably at 4°C. This ensures that the collagen is denatured and the bioink is liquid enough to print with. A temperature above 25°C induces the gelation of the collagen in the bioink, up to 50°C, where the bioink liquefies again due to the denaturation of the collagen. Therefore, bioink can be applied at values close to 2°C or higher than 50°C.

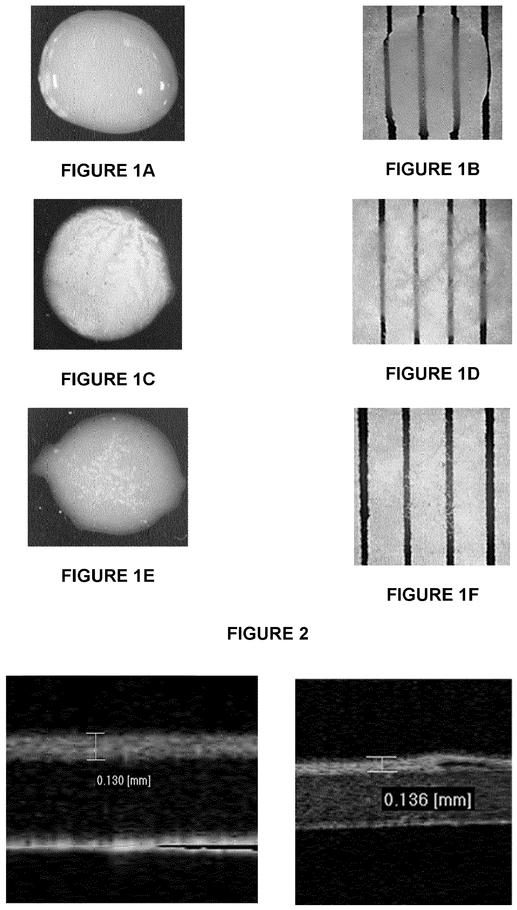

iii) Incubating the layered product obtained from stage ii at 30-40 °C to induce gelation of the collagen present in the bioink. In a preferred embodiment, it will be incubated at 37 °C. In a preferred embodiment, it will be incubated between 1 and 8 hours, more preferably for 2 hours. The establishment of the bioink at a temperature between 30 and 40°C induces its gelation. Thus, in some embodiments, the bioink is cooled to temperatures between 30 and 40 °C while, in other embodiments, the bioink is heated to temperatures between 30 and 40 °C to induce gelation. An image of the gelled construct according to this stage of the procedure can be seen in Figure 1A, and its transparency can be seen in Figure 1B.

iv) Incubating the layered product obtained from stage iii at a temperature of 20-40 °C and a humidity of 10 to 90% to induce compaction, hardening and/or controlled dehydration. The hardening and dehydration are carried out until the succession of layers comprises a size between 20 and 600 µm, with a thickness per layer between 5 and 25 µm thick. The dehydration of the construct implies a reduction in the thickness of the layers, so that during this stage the layers of the construct acquire the biomimetic size. An image of the hardened and/or dehydrated construct according to this stage of the procedure can be seen in Figure 1C, and its transparency can be seen in Figure 1D. In a preferred embodiment, humidity is at 40% and humidity control is obtained by means of a saturated solution of potassium carbonate obtained by dissolving 175g/100mL, or by other means of incubation with controlled humidity. In a preferred embodiment, the incubation lasts between 2 hours and 2 weeks, more preferably 7 days.

A thickness per layer between 5 and 25 µm thick provides a biomimetic characteristic to the construct, by resembling the size of the components of the corneal stroma. In addition, the uniformity of the diameter of the fibrils allows light to pass to the retina. It should be noted that some 3D printing methods can give rise to printing resolutions equal to those of biomimetics (between 5 and 25 µm) and therefore stage iv of dehydration and hardening can be carried out in such a way that it does not involve the reduction of the thickness per layer, or be optional, if more concentrated and viscous bioinks are used.

It is also noteworthy that not all current 3D printing methods are capable of generating constructs smaller than 20 µm per layer. In a preferred embodiment, the thickness per layer is between 20 and 25 µm.

One aspect to highlight is that both the uniformity in the thickness of the layers and the pattern formed by the angle between the direction of disposition of the bioink between layers is obtained as a result of the precision and control in the disposition of the layers provided by the technology of 3D printing in any of its forms, added to the subsequent incubation in 2 proposed phases of the construct. Therefore, other traditional ways of constructing the corneal construct other than stage ii would not be a viable way of obtaining the corneal construct. For example, the cultivation of individual layers of bioink neither allows to obtain an arrangement of the proteins in a desired order, nor their manipulation to be applied in layers. Likewise, collagen hardening with alternative methods such as incubation in N-ethyl -N'-(3-dimethylaminopropyl) carbodiimide (EDC) and N-hydroxysuccinimide (NHS), improves hardening of the construct to the detriment of its elasticity, leading to a brittle construct that can be teared when handled. Likewise, radiation with ultraviolet (UV) light, a method used in *in vivo* ophthalmology to harden the cornea in patients, produced the same results as incubation in EDC and NHS, giving rise to a construct with less elasticity without any additional advantage, as can be seen in Example 2.

It should be noted that an artificial corneal construct according to this embodiment would possess the appropriate biomimetic capabilities. In some embodiments, the construct could be hydrated, prior to its implantation in the cornea.

In some embodiments, the 1 to 25 successive layers printed by 3D printing have the same shape, such that they are vertically aligned. In other embodiments, the 1 to 25 successive layers are printed differently or vertically misaligned, such that for each point where the construct is produced in each layer, the construct can be produced on the previous layer, or it can be made on a support structure that will be removed before use. The support structure has the function of holding the produced area at the same height as the rest of the layer. Alternatively, or additionally, some layers may not fully cover the area defined by the previous layer. The impression of successive layers with different shapes or vertically not aligned better adjusts the corneal construct to the final implantation zone.

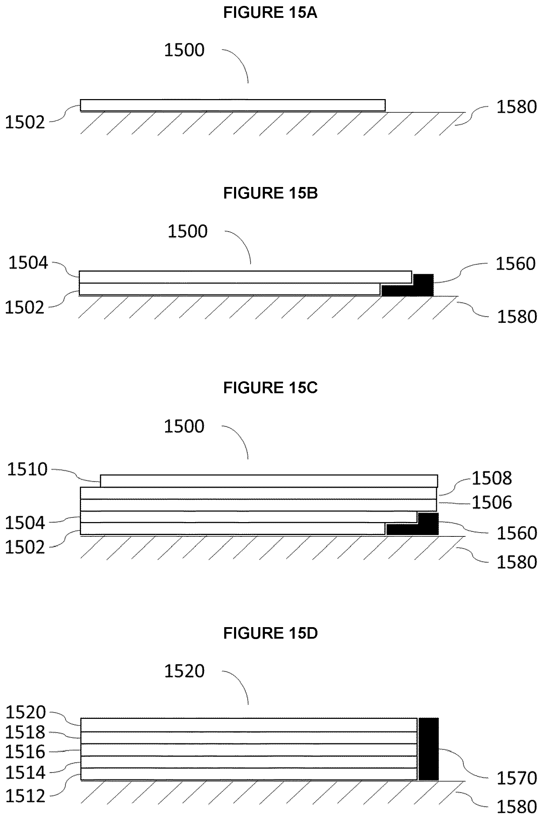

Figures 15A-C show a profile view of a corneal construct 1500 at different times in its 3D printing stage ii) according to one or more embodiments. Therefore, the different time points show the construct with a different number of printed layers. Figure 15A shows construct 1500 with only a first layer 1502 printed on a base 1580. Figure 15B shows construct 1500 once a second layer 1504 is produced on top of first layer 1502. In accordance with one or more embodiments of the invention, the direction of the collagen fibrils of layer 1504 is at an angle of 70° to 110° to the direction of the collagen fibrils of layer 1502. Figure 15C shows construct 1500 once a third layer 1506, a fourth layer 1508 and a fifth layer 1510 are produced consecutively. According to one or more embodiments of the invention, the direction of the collagen fibrils of each layer is at an angle of 70° to 110° relative to the direction of the collagen fibrils of the previous layer. In more preferred embodiments, the collagen fibrils of each layer are at a 90° angle to the direction of the collagen fibrils of the previous layer, such that alternate layers have the same printing direction. Lastly, it can be seen that layer 1510 does not completely cover the area defined by the previous layer, 1508. This allows the construct to be adapted to different needs of the recipient cornea. It is appreciated that each layer may partially or fully cover the previous layer to obtain the desired three-dimensional shape of the corneal construct.

It can also be seen that layers 1502 and 1504 are partially printed on a support structure 1560, which holds the produced area at the same height as the rest of the layer. This support structure 1560 will be discarded upon completion of the construct. In Figure 15D, a construct 1520 is shown in accordance with one or more embodiments, where a support structure 1570 is configured to laterally support each of the various layers 1522, 1524, 1526, 1528, and 1530, ensuring that the deposited bioink does not flow before being gelled. It is understood that for some types of printing, such as vat photopolymerization printing, support structures such as support structure 1560 may not be necessary to hold the produced area at the same height as the rest of the layer. Support structure 1570 may be configured to partially, or preferably fully, surround the layers of construct 1520, ensuring that the deposited bioink does not flow in any lateral direction. Support structure 1570 may be provided before, during, or after printing all layers of construct 1520.

In a preferred embodiment, the process for obtaining an artificial corneal construct also comprises the following stage.

v) Hydration of the hardened product of stage iv in a water-based solution, preferably in a buffer solution. In a preferred embodiment, the buffer solution comprises PBS, Tris, TBS or a culture medium such as "Dulbecco's Modified Eagle Medium" (DMEM) among others. In a more preferred embodiment, the buffer solution is neutral, with a pH between 6.5 and 7.5, more optionally comprising antibiotics. In a preferred embodiment, the concentration of antibiotics is 1%. An image of the rehydrated construct according to this stage of the procedure can be seen in Figure 1E, and its transparency can be seen in Figure 1F.

In Example 1, a trial in an animal model according to this embodiment is shown, where the transparency, total thickness and biocompatibility *in vitro* and *in vivo* were tested.

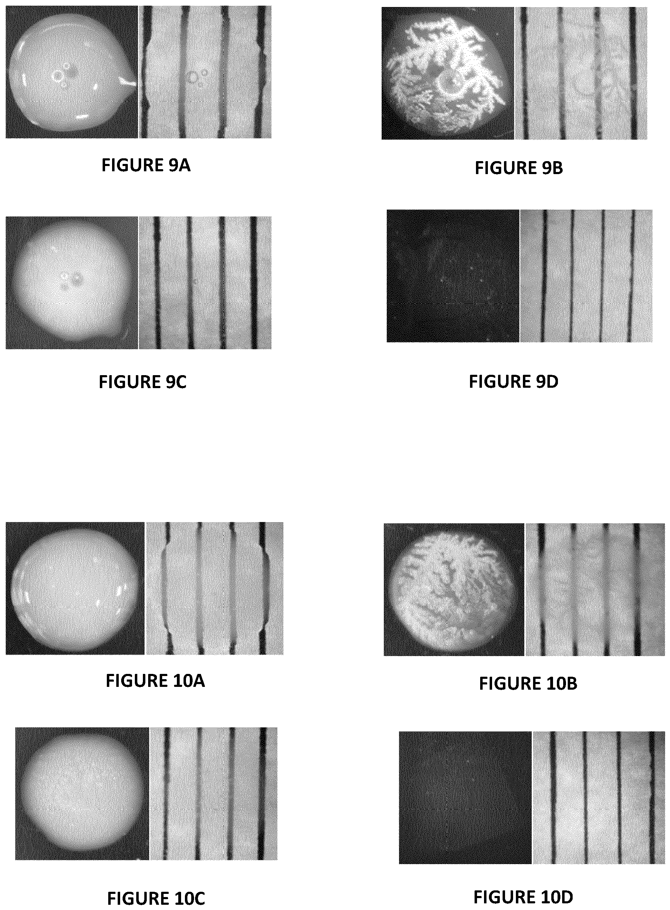

In a preferred embodiment, the composition comprises proteoglycans of corneal origin selected from the group consisting of chondroitin sulfate, dermatan sulfate and keratan sulfate or any combination thereof, said proteoglycans being found at a concentration of between 1 and 10 µg/ml. In an even more preferred embodiment, the proteoglycans are at a concentration of 9 µg/ml. Proteoglycans improve fibrillar fiber spacing and ordering by binding to collagen fibers. This is why the use of proteoglycans improves the transparency of the corneal construct as shown in Example 3 and Figures 9A-D, 10A-D, 11A-D, and 12A-D.

In a preferred embodiment, in stage ii the 3D printing is further characterized by having a diameter of the print line between 125 and 900 µm, and/or a print speed between 0.5 and 1600 mm/s, and/or a pore size of between 100 and 600 µm and/or a variation in height per layer of at least ten times the thickness per layer of stage iv. The diameter of the print stream refers to the diameter of the bioink at each point of bioink deposition; the printing speed refers to the horizontal speed at which a 3D printer produces the construct at each point, the pore size refers to the distance between adjacent printed lines of a layer that generate a free space of bioink between the printed lines within a layer, and the height variation per layer refers to the distance at which a layer occurs over the previous layer.

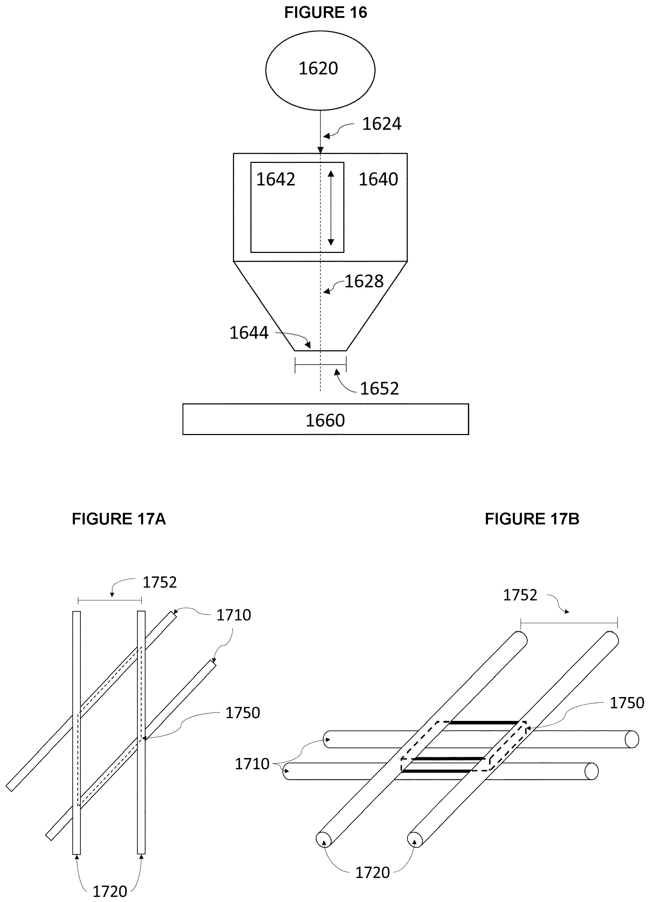

Figure 17A shows a pore size plan view of a construct according to one or more embodiments. It can be seen that the linear depositions of bioink from the lower layer 1710 and the linear depositions of bioink from the upper layer 1720 form a pore 1750 defined by the space formed by these lines of bioink. The pore size is defined by the distance 1752 between adjacent print lines. Figure 17B shows a perspective view of the pore 1750 which has a thickness equivalent to the thickness per layer of the bioink.

It is noteworthy that some characteristics of 3D printing can be obtained by different parameters depending on the type of 3D printing. Thus, the diameter of the print stream is determined by the diameter of the extrusion nozzle in extrusion printers, by the pulse frequency and size of the laser beam in laser jet printers, and by the optical fluence and laser beam size in vat photopolymerization printers. The print speed is determined by the material flow rate in extrusion printers, the pulse rate in jet printers, and the optical fluence and scan speed in vat photopolymerization printing. The pore size depends on the wettability of the substrate in jet printing and the surface coating in vat photopolymerization printing. The variation in height depends on the height of the nozzle relative to the previous layer in extruder and jet printers and the displacement of the cured layers of liquid resin from layer to layer in vat photopolymerization type printers.

In a more preferred embodiment, printing is done by extrusion and the diameter of the nozzle corresponds to the diameter of the print stream. Optionally, 3D printing is also characterized by a retraction speed of between 5 and 60 mm/s; for a running speed of between 30 and 70 mm/s and for a printing speed of between 0.5 and 700 mm/s.

Figure 16 shows a representation of an extrusion system according to one or more embodiments where printing is done by extrusion. Thus, an extruder 1640 receives a bioink 1620 through a 1624 conduit. The extruder 1640 delivers the bioink onto a surface 1660 through a nozzle 1644. The surface 1660 may be a surface initially provided for the first layer, a layer previously produced and/or a support previously added to support the layer to be printed. Nozzle 1644 has a set diameter 1652, which defines the print diameter. Likewise, extruder 1640 comprises a feed system 1642 configured to move bioink 1620 from duct 1624 through extruder 1640. The direction of flow of bioink 1620 is represented by dashed line 1628. It can be seen that feed system 1642 modifies the speed of the bioink as it passes and controls the extrusion pressure of the bioink through the nozzle 1644. In addition, it can be seen that the speed of retraction refers to the speed at which the feeding system 1642 moves the bioink in the reverse direction of the deposition, that is, vertically upwards in the case of Figure 16. This avoids the unintentional deposition of bioink during nozzle movements in which printing is not desired.

In one embodiment, 3D printing is characterized in that the printing speed also depends on the area of the layer to be printed depending on whether it is printing the perimeter, then determining the speed of the perimeter and depending on whether it is printing the filling of the layer within the perimeter, then determining the fill rate. The perimeter velocity and the filling speed are between 0.5 and 1600 mm/s. In a preferred embodiment, where the printing is done using the extrusion technique, the perimeter velocity and the fill speed are between 0.5 and 700 mm/s. It should be noted that in any embodiment of the present invention the printing speed can be defined differently in different areas of the same layer. Thus, the perimeter velocity determines the print speed when the construct is produced at the perimeter of the layer, and the fill speed determines the print speed when the construct is produced within the perimeter of each layer. In other embodiments, other types of zones are defined within the same layer with different print speeds per layer.

In a preferred embodiment, the composition in turn comprises keratocytes and/or cells capable of differentiating into "keratocytes". Cells that can differentiate into keratocytes include: corneal fibroblasts, stromal fibroblasts, fibroblasts of any origin, stromal stem cells, mesenchymal stem cells of any origin, including adipose-derived stem cells (ADSC), corneal stromal cells, multipotent cells of any origin, embryonic stem cells, pluripotent cells of any origin including induced pluripotent stem cells, mesenchymal cells of any origin, mesothelial cells, limbal stem cells, neural crest cells and/or mesenchymal stem cells. Keratocytes allow collagen fibers and extracellular matrix to be maintained by repairing the stroma in the event of inflammation and/or damage.

In another even more preferred embodiment, the keratocytes and/or cells capable of differentiating into keratocytes are present in the composition at a concentration in the range of 1 · 10 ⁵ - 6 · 10 ⁵ cells/mm³, preferably in the range of 3 · 10 ⁵ - 5 · 10 ⁵ cells/mm^{3,} with particular preference of 4 · 10 ⁵ cells/mm³. This concentration is up to 20 times higher than the native cornea, which ensures the survival of a sufficient number of keratocytes when integrating the construct, since it is a biointegration that requires a large number of keratocytes to repair the cornea weakened by various causes.

In a preferred embodiment, the collagen is 100% type I collagen. In another embodiment, the collagen is a mixture of type I, type V and/or type VI collagen in a proportion of 81% +/-30%, 17% +/- 30% and 2% +/- 30%, respectively. In another embodiment, the collagen is a mixture of type I, type V and/or type VI collagen in a proportion of 81% +/- 20%, 17% +/-20% and 2% +/- 20%, respectively. In another embodiment, the collagen is a mixture of type I, type V and/or type VI collagen in a proportion of 81% +/- 10 %, 17% +/- 10% and 2% +/- 10%, respectively. It should be noted that the proportions must be such that they add up to a unit, that is, the increase in the proportion of one type of collagen implies a reduction in the concentration of the other two types of collagen. In an even more preferred embodiment, the collagen is a mixture of 81% type I, 17% type V and/or 2% type VI collagen. The proportions of collagens type I at 81%, type V at 17% and/or type VI at 2% are "biomimetic", since they replicate the proportions of these collagens in the human cornea.

In another preferred embodiment, the thickness per final layer is +/- 50% of 20 µm thickness, and where 3D printing is characterized by presenting a fill pattern of approximately 90° keeping the bioink at a temperature between 2°C and 25°C or between 50°C and 60°C, preferably at 4°C, optionally where 3D printing is also characterized by a nozzle print stream diameter of +/- 50% of 580 µm and/or a print speed of +/- 50% of 3 mm/s, and/or a pore size of +/- 50% of 300 µm, and/or a variation of height per layer of +/- 50% of 1 mm, and/or a retraction speed of +/- 50% of 5 mm/s and/or a running speed of +/- 50% of 40 mm/s.

In another preferred embodiment, the thickness per final layer is +/- 40% of 20 µm thickness, and where 3D printing is characterized by presenting a fill pattern of approximately 90° keeping the bioink at a temperature between 2°C and 25°C or between 50°C and 60°C, preferably at 4°C, optionally where 3D printing is further characterized by a nozzle print stream diameter of +/- 40% of 580 µm, and/or a print speed of +/- 40% of 3 mm/s, and/or a pore size of +/- 40% of 300 µm, and/or a variation in height per layer of +/- 40% of 1 mm, and/or a shrinkage speed of +/- 40% of 5mm/s and/or a running speed of +/- 40% of 40mm/s.

In another preferred embodiment, the thickness per final layer is +/- 30% of 20 µm, where 3D printing is characterized by presenting a fill pattern of approximately 90° keeping the bioink at a temperature between 2°C and 25°C or between 50°C and 60°C, preferably at 4°C, optionally where 3D printing is further characterized by a nozzle print stream diameter of +/- 30% of 580 µm, and/or a print speed of +/- 30% of 3 mm/s, and/or a pore size of +/-30% of 300 µm, and/or a variation in height per layer of +/- 30% of 1 mm, and/or a shrinkage speed of +/- 30% of 5mm/s and/or a running speed of +/- 30% of 40mm/s.

In another more preferred embodiment, the thickness per final layer is +/- 20% of 20 µm thickness, and where 3D printing is characterized by presenting a fill pattern of approximately 90°, while keeping the bioink at a temperature between 2°C and 25°C or between 50°C and 60°C, preferably at 4°C, optionally where 3D printing is further characterized by a nozzle print stream diameter of +/- 20% of 580 µm, and/or a print speed of +/- 20 % of 3 mm/s, and/or a pore size of +/- 20% of 300 µm, and/or a variation in height per layer of +/- 20% of 1 mm, and/or a retraction speed of +/- 20% of 5 mm/s and/or a running speed of +/- 20% of 40 mm/s.

In another even more preferred embodiment, the thickness per final layer is +/- 10% of 20 µm thickness, and where 3D printing is characterized by presenting a fill pattern of approximately 90°, keeping the bioink at a temperature between 2°C and 25°C or between 50°C and 60°C, preferably at 4°C, optionally where 3D printing is further characterized by a nozzle print stream diameter of +/- 10% of 580 µm, and/or a print speed of +/- 10% of 3 mm/s, and/or a pore size of +/- 10% of 300 µm, and/or a variation in height per layer of +/-10% of 1 mm, and/or a speed of retraction of +/- 10% of 5 mm/s and/or a running speed of +/- 10% of 40 mm/s.

In another even more preferred embodiment, the thickness per final layer is +/- 5% of 20 µm thickness, and where 3D printing is characterized by presenting a fill pattern of approximately 90°, keeping the bioink at a temperature between 2°C and 25°C or between 50°C and 60°C, preferably at 4°C, optionally where 3D printing is further characterized by a nozzle print stream diameter of +/- 5% of 580 µm, and/or a print speed of +/ - 5% of 3 mm/s, and/or a pore size of +/- 5% of 300 µm, and/or a variation in height per layer of +/- 5% of 1 mm, and/or a speed retraction of +/- 5% of 5 mm/s and/or a running speed of +/- 5% of 40 mm/s.

In another even much more preferred embodiment, the thickness per final layer is +/- 1% of 20 µm thickness, and where 3D printing is characterized by presenting a fill pattern of approximately 90°, keeping the bioink at a temperature between 2°C and 25°C or between 50°C and 60°C, preferably at 4°C, optionally where 3D printing is further characterized by a nozzle print stream diameter of +/- 1% of 580 µm, and/or a print speed of +/- 1% of 3 mm/s, and/or a pore size of +/- 1% of 300 µm, and/or a variation in height per layer of +/- 1% of 1 mm, and/or a speed of retraction of +/- 1% of 5 mm/s and/or a running speed of +/- 1% of 40 mm/s.

In one embodiment, the thickness per layer is 20 µm thick, and where 3D printing is characterized by having a nozzle print stream diameter of 580 µm, a printing speed of 3 mm/s, a pore size of 300 µm, a filling pattern of approximately 90°, a retraction speed of 5 mm/s, a perimeter velocity of 7 mm/s, a filling speed of 7 mm/s, a running speed of 40 mm/s, and a variation in height per layer of less than 1 mm, keeping the bioink at a temperature of 4°C.

A layer thickness of 20 µm represents a biomimetic approximation of the corneal stromal collagen layers. Similarly, a 90° filling pattern is biomimetic since it replicates the arrangement of the different layers of the corneal stroma, which ensures the transparency of the construct. Keeping the temperature close to 4°C ensures that the bioink is at a temperature that does not gel.

In a preferred embodiment, where the printing is done using the extrusion technique, the 3D printing is further characterized by a retraction speed of +/- 30% of 5 mm/s, more preferably +/-10% of 5 mm/s., even more preferably 5mm/s and a running speed of +/- 30% of 40mm/s, more preferably +/- 10% of 40mm/s, even more preferably 40mm/s.

It is noteworthy that the different types of collagen (I, V and VI), proteoglycans and keratocytes can be combined in various ways. Thus, in one embodiment of the invention, the artificial corneal construct obtained by 3D printing is made up of superimposed layers of a mixture consisting of 1 to 99% type I collagen, 1 to 99% type V collagen and 1 to 99% type VI collagen, at a concentration of 0.2-80 mg/ml, optionally proteoglycans of corneal origin selected from the group consisting of chondroitin sulfate, dermatan sulfate and keratan sulfate or any combination thereof, in a range 1-10 µg/ml, and, optionally, a colony of keratocytes and/or cells capable of differentiating into keratocytes selected from the group consisting of corneal fibroblasts, stromal fibroblasts, fibroblasts of any origin, stromal stem cells, mesenchymal stem cells of any origin, including adipose-derived stem cells (ADSC), corneal stromal cells, embryonic stem cells, multipotent cells of any origin, pluripotent cells of any origin including induced pluripotent stem cells, mesenchymal cells of any origin, mesothelial cells, limbal stem cells, neural crest cells and/or mesenchymal stem cells, and combinations thereof.

In a preferred embodiment of the invention, the artificial corneal construct obtained by 3D printing is made up of superimposed layers of a mixture consisting of 81% type I collagen, 17% type V collagen and 2% type VI collagen, at a concentration of 6-10 mg/ml, and proteoglycans of corneal origin selected from the group consisting of chondroitin sulfate, dermatan sulfate and keratan sulfate or any combination thereof, in a range of 1-10 µg/ml, and, optionally, a colony of keratocytes and/or cells capable of differentiating into keratocytes selected from the group consisting of corneal fibroblasts, stromal fibroblasts, fibroblasts of any origin, stromal stem cells, mesenchymal stem cells of any origin, including stem cells derived from adipose tissue (ADSC), corneal stromal cells, embryonic stem cells, multipotent cells of any origin, pluripotent cells of any origin including induced pluripotent stem cells, mesenchymal cells of any origin, mesothelial cells, limbal stem cells, neural crest cells, and/or mesenchymal stem cells and combinations thereof.

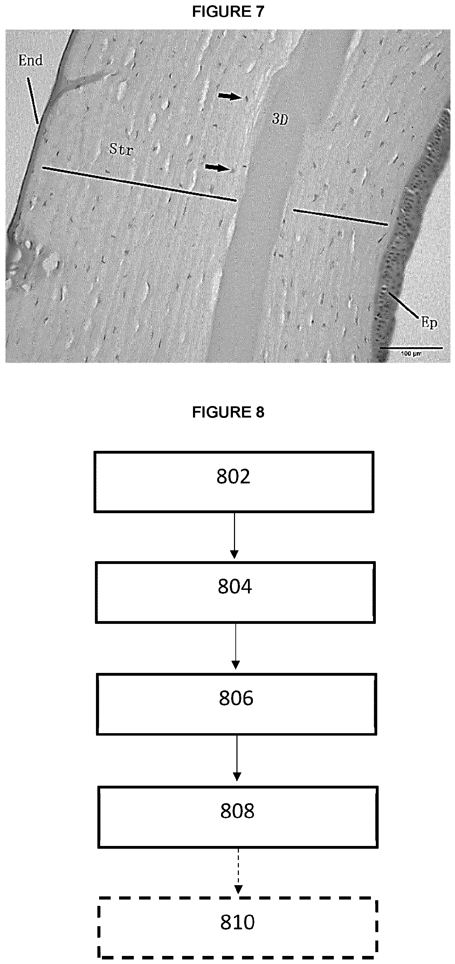

Figure 8 shows a diagram of the procedure for obtaining an artificial corneal construct according to the first aspect of the invention. The first step 802 is to provide the composition that will serve as the "bioink". This will be comprised of type I collagen or a mixture of type I, type V and/or type VI collagen at a concentration of between 0.2 and 80 mg/ml, at a pH between 3.4 and 9, and optionally proteoglycans of origin selected from the group consisting of chondroitin sulfate, dermatan sulfate and keratan sulfate or any combination thereof at a concentration of between 1 and 10 µg/ml. The second step 804 consists of printing between 1 and 25 successive layers of the composition comprising the bioink from the previous stage by 3D printing, where 3D printing is characterized by presenting an accumulated height of between 20 and 600 µm, and producing each layer to obtain collagen fibrils having a direction with an angle of 70° to 110° with respect to the direction of the collagen fibrils of the previous layer, keeping the bioink at a temperature that does not gel. Step 806 consists of incubating the layered product obtained from step 804 at 30-40 °C to induce collagen gelation. Finally, step 808 consists of incubating the product obtained from step 806 at a temperature of 20-40 °C and a humidity of 10 to 90% to induce its hardening and/or dehydration, until the succession of layers comprises a size of between 20 and 600 µm, with a thickness per layer between 5 and 25 µm thick.

Optionally, the procedure for obtaining an artificial corneal construct will comprise a final step 810 consisting of hydrating the hardened product from step iv in a neutral buffer solution optionally containing antibiotics, preferably at 1%.

In a second aspect, the invention refers to an artificial corneal construct obtainable or obtained according to any of the procedures described in the first aspect of the invention.

The artificial corneal construct obtained by 3D printing is made up of overlapping layers of a composition comprising type I collagen or a mixture of type I, type V and type VI collagen and optionally proteoglycans of corneal origin selected from the group consisting of chondroitin sulfate, dermatan sulfate and keratan sulfate or any combination thereof and, optionally, a colony of keratocytes and/or cells capable of differentiating into keratocytes selected from the group consisting of corneal fibroblasts, stromal fibroblasts, fibroblasts of any origin, stromal stem cells, stem cells mesenchymal cells of any origin, including adipose-derived stem cells (ADSC), corneal stromal cells, multipotent stem cells of any origin, embryonic stem cells, pluripotent cells of any origin including induced pluripotent stem cells, mesenchymal cells of any origin, mesothelial cells, limbal stem cells, neural crest cells, and/or mesenchymal stem cells, and combinations thereof. Preferably, the construct comprises between 1 and 25 successive layers of the composition, and is between 20 and 600 µm in size, with a thickness per layer of between 5 and 25 µm thick. Preferably, the direction of deposition of the collagen fibrils of each layer is between 70° to 110° relative to the direction of deposition of the collagen fibrils of the previous layer, more preferably at approximately 90°.

An artificial corneal construct with these characteristics can only be obtained using 3D printing technology, given the control of the size of the layers as well as the arrangement of the fibrils with a high degree of order, where the degree of order is determined by the similarity in the direction of the collagen fibrils per layer, and by the relative direction between the direction of the fibrils in each layer with respect to the adjacent layers. This is provided by the fill pattern of 3D printing, and it would not be possible to obtain this degree of order by growing a composition by other means. Since much of the biomimetics of the product is obtained from the high degree of order, it provides in turn high transparency to the product.

In an embodiment of the invention, the artificial corneal construct obtained by 3D printing is made up of superimposed layers of type I collagen, at a concentration of 0.2-80 mg/ml, and optionally proteoglycans of corneal origin selected from the group consisting in chondroitin sulfate, dermatan sulfate and keratan sulfate or any combination thereof, in a range of 1-10 µg/ml, and, optionally, a colony of keratocytes and/or cells capable of differentiating into keratocytes selected from the group consisting of corneal fibroblasts, stromal fibroblasts, fibroblasts of any origin, stromal stem cells, mesenchymal stem cells of any origin, including adipose-derived stem cells (ADSC), corneal stromal cells, embryonic stem cells, multipotent stem cells of any origin, pluripotent cells of any origin including induced pluripotent stem cells, cells mesenchymal cells of any origin, mesothelial cells, limbal stem cells, neural crest cells, and/or mesenchymal stem cells, and combinations thereof.

In another embodiment of the invention, the artificial corneal construct obtained by 3D printing is made up of overlapping layers of a mixture consisting of 1 to 99% type I collagen, 1 to 99% type V collagen and a 1 to 99% collagen type VI, at a concentration of 0.2-80 mg/ml, and proteoglycans of corneal origin selected from the group consisting of chondroitin sulfate, dermatan sulfate and keratan sulfate, in a range of 1-10 µg /ml, and, optionally, a colony of keratocytes and/or cells capable of differentiating into keratocytes selected from the group consisting of corneal fibroblasts, stromal fibroblasts, fibroblasts of any origin, stromal stem cells, mesenchymal stem cells of any origin, including cells adipose-derived stem cells (ADSC), corneal stromal cells, multipotent cells of any origin, embryonic stem cells, pluripotent cells of any origin including induced pluripotent stem cells, mesenchymal cells of any origin, mesothelial cells, limbal stem cells, neural crest cells, and/or mesenchymal stem cells, and combinations thereof.

In a preferred embodiment of the invention, the artificial corneal construct obtained by 3D printing is made up of superimposed layers of a mixture consisting of 81% +/- 10% type I collagen, 17% +/- 10% type V collagen and 2% +/- 10% type VI collagen, at a concentration of 0.2-80 mg/ml, and proteoglycans of corneal origin selected from the group consisting of chondroitin sulfate, dermatan sulfate and keratan sulfate or any combination thereof, in a range of 1-10 µg/ml, and, optionally, a colony of keratocytes and/or cells capable of differentiating into keratocytes selected from the group consisting of corneal fibroblasts, stromal fibroblasts, fibroblasts of any origin, stromal stem cells, mesenchymal stem cells of any origin, including adipose-derived stem cells (ADSC), corneal stromal cells, embryonic stem cells, multipotent stem cells of any origin, pluripotent cells of any origin including induced pluripotent stem cells, mesenchymal cells of any origin, mesothelial cells, limbal stem cells, neural crest cells and/or mesenchymal stem cells and combinations thereof.

In a preferred embodiment of the invention, the artificial corneal construct obtained by 3D printing is made up of superimposed layers of a mixture consisting of 81% type I collagen, 17% type V collagen and 2% type VI collagen, at a concentration of 0.2-80 mg/ml, and proteoglycans of corneal origin selected from the group consisting of chondroitin sulfate, dermatan sulfate and keratan sulfate or any combination thereof, in a range of 1-10 µg/ml, and, optionally, a colony of keratocytes and/or cells capable of differentiating into keratocytes selected from the group consisting of corneal fibroblasts, stromal fibroblasts, fibroblasts of any origin, stromal stem cells, mesenchymal stem cells of any origin, including derived stem cells from adipose tissue (ADSC), corneal stromal cells, multipotent cells of any origin, embryonic stem cells, multipotent stem cells of any origin including induced pluripotent stem cells, mesenchymal cells of any origin, mesothelial cells, limbal stem cells, neural crest cells, and/or mesenchymal stem cells, and combinations thereof.

In another preferred embodiment, the proteoglycans of corneal origin are present at a concentration of 9 µg/ml. In another preferred embodiment, the sample is at a concentration of 6-10 mg/ml.

Preferably, the colony of keratocytes and/or cells capable of differentiating into keratocytes is present in the artificial corneal construct of the invention in a concentration in the range of 1 · 10 ⁵ - 6 · 10 ⁵ cells/m ³, preferably in the range of 3 · 10 ⁵ - 5 · 10 ⁵ cells/mm^{3,} with special preference of 4 · 10 ⁵ cells/mm³.

In a preferred embodiment, the construct will have the shape of a rectangular parallelepiped or cylinder, where the base of the rectangular parallelepiped and the base of the cylinder have a side and diameter, respectively, between 1 and 20 mm. In a more preferred embodiment, the construct is designed for use in human corneas, and the base of the rectangular parallelepiped and the base of the cylinder have a side and diameter, respectively, between 1 and 13 mm, even more preferably between 8 and 10 mm.

In another more preferred embodiment, the construct is designed for human or veterinary use and the base of the rectangular parallelepiped and the base of the cylinder have a side and diameter, respectively, between 5 and 20 mm. The construct is characterized by a cumulative height of between 20 and 700 µm. It is noteworthy that in some embodiments the construct may have more than 25 layers and/or the layers may be more than 25 µm thick.

In a third aspect, the invention relates to the use of any of the embodiments of the artificial corneal construct described above in therapy.

In one embodiment, the construct comprises a thickness of 100 to 150 µm, for use in methods of treating ocular pathologies such as keratoconus, Terrien's marginal degeneration, pellucid marginal degeneration, Mooren's ulcer, corneal thinning, physical and chemical corneal burns., in pterygium surgery, perforation of the sclera or cornea, and strabismus; and in anterior lamellar keratoplasty and transplant treatments such as Cultured Limbal Epithelial Transplantation (CLET), Simple Limbal Epithelial Transplantation (SLET), as an amniotic membrane substitute in corneal epithelial surgeries or anterior lamellar keratoplasty, as a support or vehicle of corneal endothelium in lamellar keratoplasty surgeries, such as Descemet's membrane stripping endothelial keratoplasty (DSAEK), Descemet's membrane endothelial keratoplasty (DMEK), and/or for use on non-corneal ocular surfaces such as scleral replacement or transplant. It should be noted that in some embodiments the corneal construct can be used for other surgeries of epithelium, stroma or endothelium of the cornea.

The use of this construct allows to reinforce the cornea. It can therefore improve corneal curvature in some cases of keratoconus, replace Bowman's membrane transplantation for very advanced keratoconus, treat cases of corneal thinning, including inflammatory corneal thinning with risk of corneal perforation as well as sclera or cornea perforations, replacing the amniotic membrane that is commonly used. Its use could be extended to strabismus surgeries and the correction of ametropia by implanting sheets or segments, including presbyopia.

In another embodiment, the construct comprises a thickness of 150 to 600 µm for use in methods of treating ocular pathologies such as stromal transplantation, complete or penetrating corneal transplantation, or correction of ametropia.

The use of this construct allows to completely replace the corneal stroma. It can therefore be used in inflammatory pathologies of the corneal stroma such as severe conjunctivitis with secondary involvement of the corneal stroma: trachoma, pemphigoid, atopic keratoconjunctivitis, graft versus host disease, Stevens-Johnson syndrome, vernal keratoconjunctivitis, phlyctenular keratoconjunctivitis, blepharoconjunctivitis in all its forms, among others; in infectious keratitis, both viral (herpes simplex virus, herpes zoster virus, adenovirus), bacterial, fungal and amoebic (Acanthamoeba); in interstitial keratitis: syphilitic and non-syphilitic (Cogan syndrome, Lyme disease, tuberculosis, HSV/VHZ, sarcoidosis, and lymphomas, among others); in keratitis secondary to autoimmune diseases such as connective tissue diseases (rheumatoid arthritis, Sjogren, etc.), sarcoidosis, idiopathic (Mooren's ulcer), among others, and corneal trauma: chemical burns by acids or alkalis, inflammation associated with foreign bodies, and iatrogenesis, among others. It can also be used in non-inflammatory pathologies of the corneal stroma such as corneal dystrophies; corneal ectasias: keratoconus, keratoglobus, pellucid marginal degeneration, Terrien's marginal degeneration, post-LASIK ectasia (Laser-Assisted in Situ Keratomileusis); in corneal degeneration: Salzmann's nodular degeneration, band keratopathy, among others; in neurotrophic keratopathy; in self-inflicted keratoconjunctivitis; in neoplasms: corneal CIN, choristomas, lymphomas, among others, and in previous surgeries on the cornea with altered shape or transparency, such as radial keratotomy. Finally, it can be combined with endothelium and/or epithelium as a substitute for a complete or penetrating transplant. Its use could be extended to the potential correction of ametropia such as myopia, hyperopia, astigmatism and presbyopia.

A fourth aspect of the invention relates to a computer program. Figure 13 shows a computer program product 1300 for implementing the methods disclosed herein, which may be implemented in software, hardware, or a combination thereof. The computer program 1300 may be stored in a 3D printer memory, and/or optionally in the memory of a media formulation mixer or incubation chamber or may be stored remotely, for example, on a remote server communicatively connected to a 3D printer, and optionally to an incubation chamber or media formulation mixer. The computer program product 1300 is communicatively connected to the electronics of the 3D printer, and optionally those of the media formulation mixer and the "incubation chamber". Computer program 1300 comprises computer-readable instructions that, when executed by a processor, cause the processor to execute one or more of the modules described below.

The processor may comprise one or more processing units, such as a microprocessor, a GPU, a CPU, and a multicore processor or the like. Similarly, memory may comprise one or more volatile or non-volatile memory devices, such as DRAM, SRAM, flash memory, read-only memory, ferroelectric RAM, hard disk drives, floppy disks, magnetic tapes, optical disks, or the like. The controller may be implemented in software (a computer program), hardware (a physical device), or any combination for the purpose of executing the sequences of operation disclosed in this document.

Computer program 1300 may be configured to perform any of the steps in the method described in connection with Figure 8.

Optionally, in embodiments where the computer program is connected to a media formulation mixer, the computer program comprises a sample selector module, 1302, that is configured to select a given percentage of type I, type V, and type VI collagen, and proteoglycans from particular solutions of type I, type V and type VI collagen and proteoglycans. Therefore, the sample selector module 1302 is capable of, from each type of component, obtaining a composition with desired proportions of type I, type V and type VI collagen and proteoglycans. The ratios may be stored in memory according to preset values or entered by a user through an interface. In some embodiments, the particular solutions of type I, type V, and type VI collagen and proteoglycans are isolated, while in other embodiments the solutions comprise pre-established mixtures of these elements. Thus, it is possible that a solution includes, for example, collagen type I at 81%, type V at 17%, and type VI at 2%, and given a preselection of this type of collagen mixture, the sample selector module 1302, select the selection that contains this sample. Other solutions may comprise combinations of collagens, proteoglycans and/or keratocytes in different proportions. Alternatively, the 3D printer can be supplied with a pre-prepared bioink.

Therefore, the sampler module 1302 can prepare a composition comprising type I collagen or a mixture of type I, type V and/or type VI collagen at a concentration between 0.2 and 80 mg/mL, at a pH between 3.4 and 9 and where the composition optionally comprises proteoglycans of corneal origin selected from the group consisting of chondroitin sulfate, dermatan sulfate and keratan sulfate or any combination thereof at a concentration of between 1 and 10 µg/ml.

The computer program product comprises a 3D printing module 1304, configured to control the element that produces the layers of the construct. Given values for number of layers, stream diameter, print speed per layer, pore size, fill pattern, and height per layer, controls the element that produces the layers of the construct in three-dimensional space to form the 3D construct. The values for the number of layers, stream diameter, print speed per layer, pore size, fill pattern, and height per layer may be stored in memory or entered by a user. Similarly, predefined combinations of the various parameters may be stored in memory, and the user select one of the predefined combinations.

The computer program product optionally comprises a layer selector module 1306, configured to define the number of layers of the construct to be printed given a maximum thickness of the construct, the layer selector module 1306 can define the number of layers based on a thickness desired thickness per layer and provide the value to the 3D printing module 1304. The desired thickness per layer may be stored in memory or entered by a user.

Optionally, in embodiments where the 3D printer comprises a thermometer and means for heating and/or cooling the bioink, the computer program product comprises a bioink temperature selector module 1308, configured to measure the temperature of the bioink, determine if it is at the desired temperature, and modify it if it is different by activating the means to heat and/or cool the bioink. The desired bioink temperature can be stored in memory, entered by a user, or selected from a list of predefined values.

Therefore, the 3D printing module 1304 can print between 1 and 25 layers of a composition by 3D printing, where the composition in bioink form comprises type I collagen or a mixture of type I, type V and/or type VI collagens, at a concentration between 0.2 and 80 mg/ml, at a pH between 3.4 and 9 and where the composition optionally comprises proteoglycans of corneal origin selected from the group consisting of chondroitin sulfate, dermatan sulfate and keratan sulfate or any combination thereof at a concentration between 1 and 10 µg/ml; and where the 3D printing is characterized by presenting an accumulated height of between 200 and 6000 µm, and producing each layer to obtain collagen fibrils having a direction with an angle of 70° to 110° with respect to the direction of the collagen fibrils of the layer above, keeping the bioink at a temperature that does not gel. Optionally, the 3D printing module 1304 can print the 1 to 25 successive layers with different shapes or vertically not aligned, in such a way that for each point where the construct is produced in each layer, the construct can be produced on the previous layer, or it can be made on a support structure that will be removed before use. The shape and alignment of each layer can be stored in memory, entered by a user, or selected from a list of predefined values.

It is noteworthy that in other non-corneal artificial constructs, the desired transparency may be different. Thus, alternatively, when the artificial construct produced requires other transparencies, the 3D printing module 1304 can print between 1 and 25 layers of the previously mentioned composition, where the 3D printing can be characterized by producing each layer to obtain collagen fibrils, having a direction with an angle between 0° and 359° with respect to the direction of the collagen fibrils of the previous layer. For example, when the artificial construct is made for sclera, 3D printing is characterized by producing each layer to obtain collagen fibrils having a direction with an angle between 315° and 45° with respect to the direction of the collagen fibrils of the previous layer.

Optionally, in embodiments where the computer program is connected to an incubation chamber, the computer program product also comprises an incubation temperature selector module 1310, configured to measure the temperature in the incubation module, determine if it is to the desired temperature and modify the temperature of the incubation module if it is different. The desired incubation module temperature can be stored in memory, entered by a user, or selected from a list of predefined values.

Optionally, in embodiments where the computer program is connected to an incubation chamber, the computer program product also comprises an incubation humidity selector module 1312, configured to measure the humidity in the incubation module, determine if it has the desired humidity, and modify the humidity of the incubation module if it is different from the desired one. The desired incubation module temperature can be stored in memory, entered by a user, or selected from a list of predefined values.

It should be noted that the incubation temperature 1310 and incubation humidity 1312 selector modules can work together, and therefore configure the incubation chamber to incubate the product resulting from layered printing at 30-40 °C to induce gelation of the collagen. Likewise, they can configure the incubation chamber to incubate the gelled layered product at a temperature of 20-40 °C and a humidity of 10 to 90% to induce its hardening and / or controlled dehydration until the succession of layers comprises a size of between 20 and 600 µm with a thickness per layer of between 5 and 25 µm. The determination of the moment in which the succession of layers comprises a size between 20 and 600 µm, with a thickness per layer of between 5 and 25 µm thick, can be done using an equivalence table or a mathematical formula obtained through previous tests with similar constructs, where for a given number of layers, stream diameter, printing speed per layer, pore size, fill pattern and height per layer, a curing time is given. The equivalence table and/or the mathematical formula may be stored in memory.

Optionally, in embodiments where the computer program is connected to an incubation chamber, the computer program product also comprises a solution module 1314, configured to introduce and withdraw a solution into the incubation chamber to allow hardening and/or controlled dehydration of the incubated construct. Solution introduction and withdrawal times may be stored in memory or entered by a user. The injection module can therefore configure the injection chamber to further hydrate the hardened product from stage iv in a neutral buffered solution optionally comprising antibiotics, preferably at 1%.

The computer program product comprises a time phase detection module 1314, configured to receive information from the electronic components of the 3D printer and determine when it starts and stops working. In embodiments where the computer program is connected to a media formulation mixer, the phase detection module 1314, in turn, is capable of receiving information from the electronic components of the media formulation mixer and determining the moment in which it begins and ends working. Likewise, in the embodiments where the computer program is connected to an incubation chamber, the phase detection module 1314, in turn, is capable of receiving information from the electronic components of the incubation chamber and determining the moment in which it starts and stops working. Therefore, the time phase detection module 1314 is able to communicate with the 3D printer, optionally a media formulation mixer and optionally an incubation chamber, and determine the start of a phase after the completion of a previous phase. In this way, the time phase detection module 1314 can allow manual or automatic synchronization between the 3D printer, a media formulation mixer, and/or an incubation chamber.

### Examples

### 3D printing parameters

i. Number of layers: 1 to 25, optimal 5 for a thickness of 130 µm and 20 (4 stacks of 5 layers each) for a thickness of 400 µm
ii. Layer thickness (layer height): 200 to 250 µm, optimum 200 µm
iii. Flow rate: from 0.5 to 4 mm/s, optimal 3 mm/s
iv. Nozzle diameter: 125 to 900 µm, optimal 580 µm (20G)
v. Pore size: 100 to 5600 µm, optimal 300 µm
vi. Load pattern: diagonal from 45° to 90°, optimal at 90°
vii. Retraction speed: from 5 to 60 mm/s, optimal 5 mm/s
viii. Perimeter: yes
ix. Perimeter velocity: from 4 to 7 mm/s, optimal 7 mm/s
x. Fill rate: 4 to 7 mm/s, optimal 7 mm/s
xi. Running speed: 30 to 70 mm/s, optimal 40 mm/s
xii. Height variation: 1 to 5 mm, optimal 1 mm
xiii. Bioink temperature from 2°C to 30°C, optimal 4°C

### Example 1: Tests in animal models

For the following trials, adult rabbits were used, which were implanted with a partial artificial corneal construct of the invention with a diameter of 6 mm and a thickness or height of 120 µm.

To do this, under a surgical microscope and after a superior corneal paralimbar preincision of 5 mm length and 200 µm depth performed at 90°, an intrastromal corneal pocket was created following the 200 µm plane of depth and 7 mm in diameter with an ophthalmic "crescent" type knife in the central cornea, where to accommodate the 3D printed stroma, which was introduced folded to shortly after, once inside the pocket in the rabbit cornea, be unfolded and centered. The incision was then closed with two 10/0 nylon sutures. The printed construct was introduced only into one eye of each animal (left eye) for the experiment and the contralateral (right) eye did not undergo surgery and served as a control. After surgery, the appropriate postsurgical treatment (0.3% topical ciprofloxacin, 0.5% cyclopentolate hydrochloride, and subcutaneous buprenorphine) was instilled twice a day for four days and the rabbits were monitored twice a week, being sacrificed after four months.

### Transparency and thickness in vitro

Different photographs of a printed product consisting of type I collagen (10 mg/ml) and glycosaminoglycans (9 µg/ml) were obtained in the different stages of incubation and rehydration of the process described above, on both a black background and a back-lit background of vertical lines (standard methods in ophthalmology), as shown in Figure 1. Figures 1A and 1B show the construct after its gelation. Figures 1C and 1D show the construct after dehydration. Figures 1E and 1F show the construct after rehydration. The transparency achieved is similar to the native cornea's.

The thickness of the product was also measured by OCT tomography (see Figure 2), resulting in a thickness of 0.130 - 0.136 mm. The thickness achieved was as expected, similar to the decellularized corneal lamina mentioned in the introduction, which was previously tested in studies on rabbits and in clinical trials on patients with keratoconus.

### In vitro biocompatibility

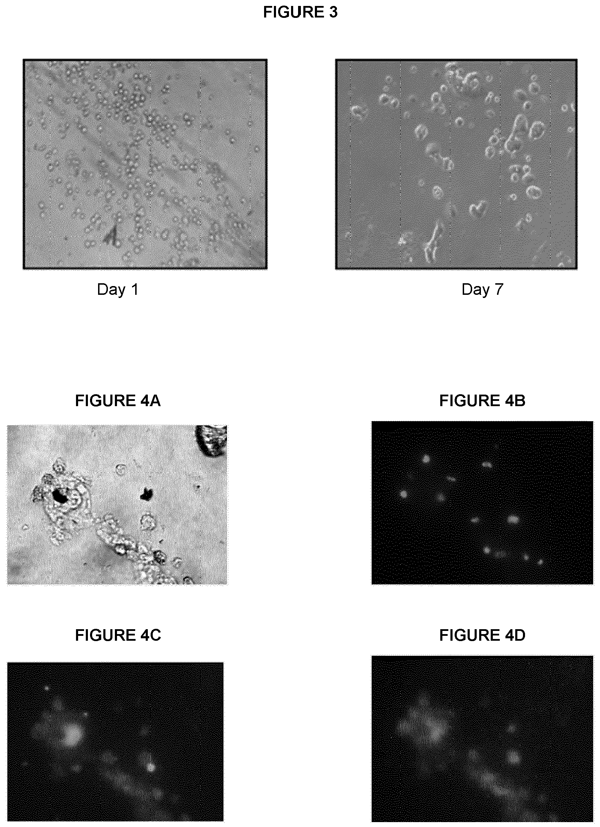

Figures 3 and 4 are photographs showing the *in vitro* biocompatibility of the artificial corneal construct of the invention with keratocytes on days 1 and 7 of culture (Figure 3, phase contrast microscopy) and the targeted differentiation of adipose tissue-derived stem cells into keratocytes (Figures 4A -D) phase contrast (Figure 4A), by staining the nucleus with DAPI (4',6'-diamidino-2-phenylindole) and fluorescence (Figure 4B), showing the secretion of keratocan (Figure 4C) and collagen IV (Figure 4D) by immunofluorescence at 21 days of differentiation. Biocompatibility with keratocytes and optimal targeted differentiation of stem cells derived from human adipose tissue were verified.

### Transparency, biosafety and in vivo biocompatibility

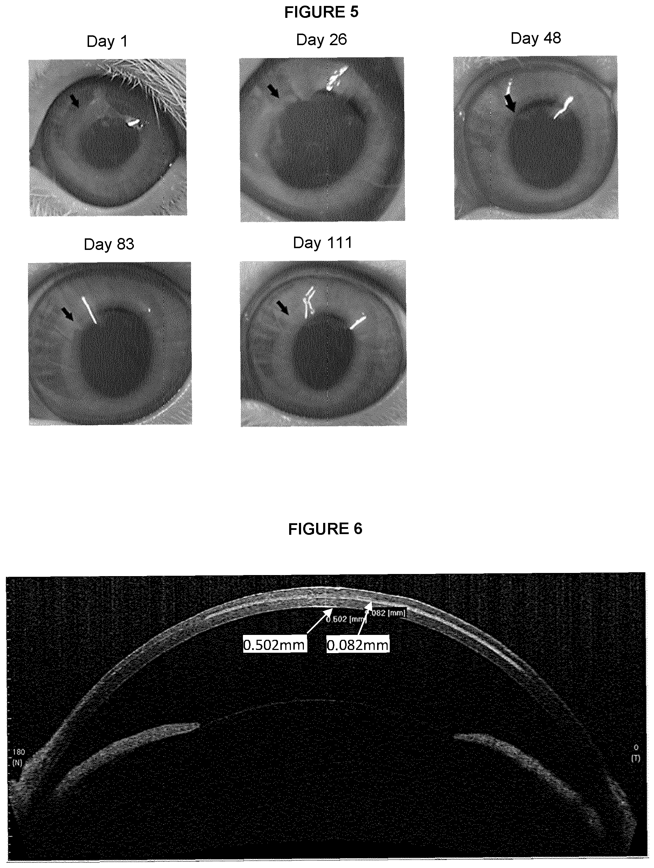

To check the transparency in a macroscopic way, a study was carried out during 118 days, including the day of sacrifice. The study consisted of monitoring the evolution of the graft by means of sequential macroscopic photographic shots every 7 days (Figure 5). The objective was to check the biocompatibility of the graft and its possible improvement in terms of transparency and refraction.

Once the experimental and control eyes were enucleated, an optical coherence tomography (OCT) study was performed. This test uses low-coherence light beams (1280-1300 nm) and obtains high-resolution images or slices non-invasively. The use of the anterior segment module (OCT-SA) allows the study of the cornea, including thickness, curvature, and elevation maps of the anterior and superior faces, as well as the measurement of structures such as the anterior chamber (Figure 6). To complete the study, we included the samples in paraffin and a subsequent hematoxylin-eosin staining was performed on histological sections, which allowed us to carry out a histological study of the transplanted corneas (Figure 7).

Macroscopic images of rabbit eyes transplanted with the artificial construct (100 to 120 µm thick) described here were obtained. As seen in Figure 5, no signs of inflammation, vascularization or rejection were observed at different weeks and up to 4 months after transplantation. An optical coherence tomography (OCT) image was also obtained showing complete biointegration of the imprinted transplant at four months (see Figure 6). In Figure 6, it can be seen that the total thickness of the cornea in the central section was 0.502mm, where the construct, in lighter color, represents 0.082mm. Finally, Figure 7 shows a histological image of the cornea showing the printed transplant (3D) biointegrated in the corneal stroma (str), with no signs of inflammation, vascularization, immunological reaction, no retroprosthetic formations, no fibrosis or activation of the host keratocytes (arrows), observing the normal functioning of the endothelial cells without histopathological signs of edema or rejection.

### Example 2: Variations of the procedure with negative result

A second test was carried out in order to determine the feasibility of hardening methods in the state of the art.

Given two constructs printed with the aforementioned 3D printing parameters, gelled according to stage iii) of the procedure for obtaining an artificial corneal construct by 3D printing described for 2h at 37°C, two methods commonly used for collagen hardening and in vivo corneas were proposed, respectively.

One procedure consisted of incubating the construct in 20mM EDC (N-ethyl-N'- (3-dimethylaminopropyl) carbodiimide) and 10mM NHS (N-hydroxysuccinimide) in a MOPS buffer solution at pH 7.4 to ensure cell viability, at a temperature of 37°C. EDC/NHS is used in other applications for collagen hardening. Incubation was extended for 24 hours, with analysis of the construct at 2, 4 and 24 hours.

The other procedure consisted of irradiating the construct with ultraviolet (UV) light in cycles from 28.8 Jul/m ² to 172.8 Jul/m ², applying up to 6 cycles, each cycle lasting 6 minutes. UV light is used in ophthalmology *in vivo* to harden the cornea in patients. The construct was analyzed after each cycle.

### Construct - independent analysis

Two identical constructs gelled according to stage iii of the procedure were exposed to EDC/NHS incubation and UV light, respectively.

The construct treated with EDC/NHS improved the hardness of the construct to the detriment of its elasticity, and it was observed that with the passage of time in incubation, the construct became more brittle, leading to its tearing more easily. Therefore, their strength and biomechanics were reduced. On the other hand, the construct treated with UV light did not see its transparency affected, and its hardness increased with the passing of the cycles in a similar way to the previously described chemical treatment, giving rise to an equally hard but easily teared construct.

It was observed, therefore, that the usual procedures for hardening collagen and cornea do not give the same results as the hardening and dehydration process proposed in stage IV, since the construct is weaker due to its lower elasticity.

### Combined analysis

Next, the possibility of combining the proposed hardening and dehydration method in stage iv of the procedure for 7 days at 40% humidity together with incubation with EDC/NHS and/or exposure to UV light was tested.

The construct dehydrated and hardened according to stage iv and incubated with EDC/NHS gave rise to a hard but tearable brittle construct. The construct dehydrated and hardened according to process stage iv and exposed to UV light cycles did not show any improvement over the construct treated only according to process stage iv.

Finally, the construct dehydrated and hardened according to stage iv, incubated with EDC/NHS and exposed to UV light cycles, obtained a slight improvement in the strength of the construct.

It was observed that only the combination of the 3 methods is capable of providing a slight improvement in the mechanical characteristics of the construct.

### Example 3: Process Optimizations

A third trial was directed towards optimizing the bioink composition and process parameters with the goal of obtaining optimal in vitro transparency. To analyze the transparency of the different constructs produced by the different bioinks, different photographs were obtained at the different stages of incubation and rehydration of the process described above on a black background and a back-lit background of vertical lines (standard methods in ophthalmology).

Four combinations of bioink components were tested in the analysis. First, a composition consisting of type I collagen (8mg/ mL). The second bioink included, in addition to type I collagen, GAG proteoglycans at a concentration of 1µg/ml. A third bioink added the proteoglycans to type I collagen in a higher concentration than the second, at 9µg/ml. Finally, a biomimetic mixture of collagens (81% type I collagen (10mg/ml), 17% type VI collagen (0.62mg/ml), 2% type V collagen (0.71 mg/ml)), together with GAG proteoglycans (9µg/ml).

All corneal constructs followed the same impression and curing process. The impression consisted of the application of 5 layers, with a perpendicular filling pattern (90°). The gelation treatment consisted of incubation for 2h at 37°C. Hardening, compaction and dehydration were applied for 1 week by incubation at 40% relative humidity. Rehydration was applied by immersing the construct in PBS solution.

The first bioink, formed only by type I collagen, gave rise to a non-optimal transparency as can be seen in Figures 9A to 9D. In Figure 9A the construct can be seen after gelation on the two backgrounds. In Figure 9B, the construct can be seen after the curing process (hardening and dehydration). Figure 9C shows the construct after the rehydration process. Figure 9D shows the construct after its inclusion in glycerol, simulating the *in vivo* corneal environment. It was noted that while there are no severe image distortions, the transparency of the image is not optimal.

The second bioink, made up of type I collagen and GAG proteoglycans (1µg/ml), gave rise to non-optimal transparency as can be seen in Figures 10A to 10D. Figure 10A shows the construct after gelation on the two backgrounds. In Figure 10B, the construct can be seen after the curing process (hardening and dehydration). Figure 10C shows the construct after the rehydration process. Figure 10D shows the construct after its inclusion in glycerol, simulating the *in vivo* corneal environment. It was observed that, although the transparency improves with respect to the first bioink, it is still not optimal.

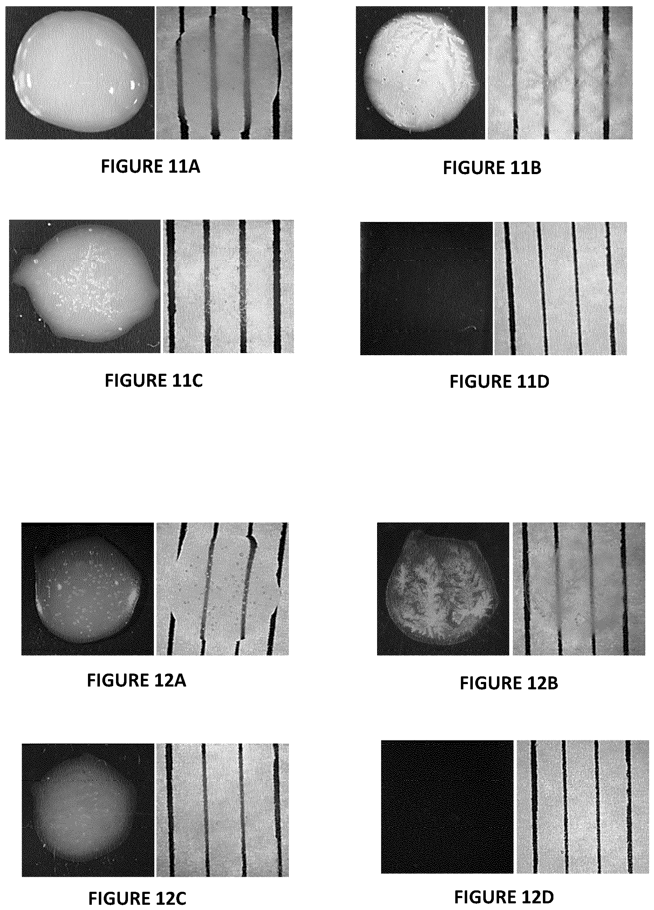

The third bioink, made up of type I collagen and GAG proteoglycans (9µg/ml), gave rise to optimal transparency as can be seen in Figures 11A to 11D. In Figure 11A the construct can be seen after gelation on the two backgrounds. In Figure 11B, the construct is seen after the curing process. Figure 11C shows the construct after the rehydration process. Figure 11D shows the construct after its inclusion in glycerol, simulating the *in vivo* corneal environment. It was observed that the transparency obtained in the implantation (Figure 11D) is almost perfect, giving rise to an optimal composition in terms of transparency.

Finally, the fourth bioink, made up of collagens (81% type I collagen (10mg/ml), 17% type VI collagen (0.62mg/ml), 2% type V collagen (0.71mg/ml)), together with GAG proteoglycans (9 µg/ml), gave rise to a still optimal transparency as can be seen in Figures 12A to 12D. In Figure 12A the construct can be seen after gelation on the two backgrounds. In Figure 12B, the construct can be seen after the curing process. Figure 12C shows the construct after the rehydration process. Figure 12D shows the construct after its inclusion in glycerol, simulating the *in vivo* corneal environment. It can be seen that the transparency obtained in the implantation (Figure 11D) is perfect, being unappreciable in neither of the 2 backgrounds. It can be said that this biomimetic composition is the most optimal of all.

Therefore, it was observed that the presence of proteoglycans increases the transparency of the construct, and that the use of a biomimetic collagen combination increases transparency even more, until construct is imperceptible.

## Claims

1. Procedure for obtaining an artificial corneal construct by three-dimensional (3D) printing, comprising the following stages:
i. providing a composition in the form of bioink comprising type I collagen or a mixture of type I, type V and/or type VI collagen at a concentration between 0.2 and 80 mg/ml, at a pH between 3.4 and 9 and where the composition optionally comprises proteoglycans of corneal origin selected from the group consisting of chondroitin sulfate, dermatan sulfate and keratan sulfate or any combination thereof, at a concentration between 1 and 10 µg/ml;
ii. printing between 1 and 25 successive layers of the composition comprising the bioink from the previous stage by means of 3D printing, where 3D printing is **characterized by** presenting an accumulated height of between 200 and 6000 µm, and producing each layer to obtain collagen fibrils having a direction with an angle of 70° to 110° with respect to the direction of the collagen fibrils of the previous layer, keeping the bioink at a temperature that does not gel;
iii. incubating the layered product obtained from stage ii at 30-40 °C to induce collagen gelation; and
iv. incubating the layered product obtained from stage iii at a temperature of 20-40°C and a humidity of 10 to 90% to induce its compaction, hardening and/or controlled dehydration until the succession of layers comprises a size between 20 and 600 µm, with a thickness per layer between 20 and 25 µm thick.

2. Procedure for obtaining according to claim 1, further comprising the hydration of the hardened product of stage iv in a water-based solution, preferably in a buffer solution.

3. Procedure according to any of claims 1 or 2, wherein stage ii the 3D printing is further **characterized by** having a diameter of the printing stream between 125 and 900 µm, and/or a printing speed between 0.5 and 1600 mm /s, and/or a pore size between 100 and 600 µm and/or a variation in height per layer of at least ten times the thickness per layer of stage iv;
wherein the diameter of the printing stream is understood as the diameter of the bioink at each point of bioink deposition; wherein the printing speed is understood as the horizontal speed at which a 3D printer produces the construct at each point; wherein the pore size is understood as the distance between adjacent printed lines of a layer that generate a free space of bioink between the lines printed within a layer, and wherein the variation in height per layer is understood as the distance at which one layer occurs over the previous layer.

4. Procedure according to claim 3, wherein the 3D printing is carried out by extrusion and where the diameter of the nozzle corresponds to the diameter of the printing stream, optionally where the 3D printing is also **characterized by** a retraction speed of between 5 and 60 mm/s; and/or by a running speed of between 30 and 70 mm/s and/or by a printing speed of between 0.5 and 700 mm/s.

5. Procedure according to any of claims 1 to 4, wherein the composition further comprises keratocytes and/or cells capable of differentiating to keratocytes such as corneal fibroblasts, stromal fibroblasts, fibroblasts of any origin, stromal stem cells, mesenchymal stem cells of any origin, including adipose-derived stem cells (ADSC), corneal stromal cells, multipotent stem cells of any origin, embryonic stem cells, pluripotent cells of any origin including induced pluripotent stem cells, mesenchymal cells of any origin, mesothelial cells, limbal stem cells, neural crest cells, and/or mesenchymal stem cells.

6. Procedure according to claim 5, wherein the keratocytes and/or cells capable of differentiating into keratocytes are present in the composition at a concentration in the range of 1 · 10 ⁵- 6 · 10 ⁵ cells/mm^{3,} preferably in the range of 3 · 10 ⁵ - 5 · 10 ⁵ cells/mm³.

7. Procedure according to any of the preceding claims, wherein the collagen is 100% type I collagen or a mixture of type I, type V and/or type VI collagen in a proportion of 81% +/- 10%, 17% +/- 10% and 2% +/- 10%, respectively.

8. Procedure according to any of the above claims, wherein the thickness per final layer is +/- 30% with a thickness of 20 µm, and where the 3D printing is **characterized by** presenting a diagonal load pattern of approximately 90°, keeping the bioink at a temperature between 2°C and 25°C or between 50°C and 60°C, preferably at 4°C, optionally where 3D printing is further **characterized by** a nozzle print stream diameter of +/- 30% of 580 µm, a printing speed of +/- 30 % of 3 mm/s, a pore size of +/- 30% of 300 µm, a variation in height per layer of +/- 30% of 1 mm, a retraction speed of +/- 30% of 5 mm /s and/or a running speed of +/- 30% of 40 mm/s.

9. Procedure according to any of claims 1 to 6, wherein the thickness per final layer is +/-10% with a thickness of 20 µm, and where the 3D printing is **characterized by** presenting a diagonal loading pattern of approximately 90°, keeping the bioink at a temperature between 2°C and 25°C or between 50°C and 60°C, preferably at 4°C, optionally where 3D printing is also **characterized by** a nozzle print stream diameter of +/- 10% of 580 microns, a printing speed of +/- - 10% of 3 mm/s, a pore size of +/- 10% of 300 µm, a variation in height per layer of +/- 10% of 1 mm, a retraction speed of +/- 30% of 5 mm/s and/or a running speed of +/- 30% of 40 mm/s.

10. Procedure according to any of the preceding claims, wherein the composition comprises proteoglycans of corneal origin selected from the group consisting of chondroitin sulfate, dermatan sulfate and keratan sulfate or any combination thereof, said proteoglycans being found at a concentration of between 1 and 10 µg/ml, preferably at 9 µg/ml.

11. Procedure according to any of the preceding claims, wherein the method comprises providing a support structure to support each of the layers to maintain the shape of the printed layers in stage ii.

12. Artificial corneal construct obtainable or obtained according to any of claims 1 to 11, preferably in the form of a rectangular parallelepiped or cylinder, wherein the base of the rectangular parallelepiped and the base of the cylinder have one side and diameter, respectively between 1 and 20 mm, more preferably between 1 and 13 mm, even more preferably between 8 and 10mm.

13. Artificial corneal construct according to claim 12, for its use in therapy.

14. Artificial corneal construct according to claim 12:
wherein the construct comprises a thickness of 100 to 150 µm, for use in methods of treating ocular pathologies such as keratoconus, Terrien's marginal degeneration, pellucid marginal degeneration, Mooren's ulcer, corneal thinning, corneal burns, in pterygium surgery, perforation of the sclera or cornea, and strabismus; in limbus transplant treatments such as CLET and SLET, as a substitute for the amniotic membrane in corneal epithelial surgeries or anterior lamellar keratoplasty and as a support or vehicle for the corneal endothelium in keratoplasty surgeries such as DSAEK and/or DMEK;
or wherein the construct comprises a thickness of 150 to 600 µm, for use in methods of treating ocular pathologies such as stromal transplant, complete or penetrating corneal transplant or correction of ametropia.

15. A computer program, connected to a 3D printer comprising computer readable instructions which, when executed by a processor, cause the processor to execute the step of printing between 1 and 25 successive layers of a composition by 3D printing, wherein the composition in the form of bioink comprises type I collagen or a mixture of type I, type V and/or type VI collagen at a concentration between 0.2 and 80 mg/ml, at a pH between 3.4 and 9 and wherein the composition optionally comprises proteoglycans of corneal origin selected from the group consisting of chondroitin sulfate, dermatan sulfate and keratan sulfate at a concentration between 1 and 10 µg/ml; and wherein the 3D printing is **characterized by** presenting an accumulated height of between 200 and 6000 µm, and producing each layer to obtain collagen fibrils having a direction with an angle of 70° to 110° with respect to the direction of the collagen fibrils of the layer above, keeping the bioink at a temperature that does not gel.
